# EUROPEAN PATENT APPLICATION

(11) **EP 4 343 004 A2**
(43) Date of publication of application: **27.03.2024**
(21) Application number: 23216708.0
(22) Date of filing: 18.10.2021
(51) Int. Cl.: C12Q 1/6886

(54) **GERMLINE BIOMARKERS OF CLINICAL RESPONSE AND BENEFIT TO IMMUNE CHECKPOINT INHIBITOR THERAPY**

(30) Priority: 19.10.2020 US 202063093616 P; 13.08.2021 US 202163232785 P
(62) Divisional of application: 21805792.5
(71) Applicant: Dana-Farber Cancer Institute, Inc., Boston, MA 02115-5450 (US)
(72) Inventor: GUSEV, Alexander, Cambridge, 02138 (US); CHOUEIRI, Toni, K., Westwood, 02090 (US); FREEDMAN, Matthew, Needham, 02492 (US)
(74) Representative: Hoefer & Partner Patentanwälte mbB

(57) **Abstract**

The present invention is based on the identification of novel metabolite biomarkers predictive of development of immune-related adverse events when receiving anti-immune checkpoint therapies.

## Description

### Related Applications

This application claims the benefit of priority to U.S. Provisional Patent Application No. 63/093,616, filed on 19 October 2020, and U.S. Provisional Patent Application No. 63/232,785, filed on 13 August 2021, the entire contents of each of said applications are incorporated herein in their entirety by this reference.

### Statement of Rights

This invention was made with government support under grant number R01CA227237 awarded by The National Institutes of Health. The government has certain rights in the invention.

### Background of the Invention

Tumors co-opt immune-checkpoint pathways in order to avoid elimination by the host immune system (Pardoll (2012) Nat. Rev. Cancer 12:252-264). Thus, immune checkpoint therapies can yield durable responses and long-lasting survival benefit across some cancer types (Topalian et al. (2015) Cancer Cell 27:450-461). Indeed, checkpoint therapies have been approved for use in metastatic melanoma, non-small cell lung cancer, bladder cancer, and renal cell carcinoma, including as a first-line therapy for non-small cell lung cancer.

However, many subjects develop immune related adverse events (irAE), which are often severe, leading to hospitalization, mandatory discontinuation of treatment, or even death.

It is presently unclear what factors, if any, determine whether a subject receiving immune checkpoint inhibitors will develop irAE or not. Thus, there is a need to identify biomarkers predictive of development of irAE for patients receiving immune checkpoint therapy.

### Summary of the Invention

The present invention is based, at least in part, on the discovery that certain germline variations (*e.g.,* mutations, SNPs, and the like) can be specific biomarkers for prognostic and predictive development of irAE in patients receiving immune checkpoint therapy (*e.g.,* PD-1 inhibitors). Accordingly, the present invention relates, in part, to methods for stratifying patients and predicting development if irAE in a subject having cancer and receiving immune checkpoint therapy based upon a determination and analysis of amounts of such biomarkers, compared to a control. In addition, such analyses can be used to provide useful anti-immune checkpoint treatment regimens (*e.g.,* based on predictions of the development of irAE).

In an aspect of the invention, methods of identifying the likelihood of development of immune-related adverse events (irAE) in a subject due to immune checkpoint therapy include a) obtaining or providing a subject sample from a subject having cancer; b) measuring the amount of at least one germline biomarker listed in Table 1 in the subject sample; and c) comparing said amount of the at least one germline biomarker listed in Table 1 to a control. In these methods, the presence of or a significantly increased amount of the at least one germline biomarker listed in Table 1 in the subject sample, relative to the control, identifies the development of irAE due to immune checkpoint therapy as being more likely; and wherein the absence of or a significantly decreased amount of the at least one germline biomarker listed in Table 1 in the subject sample, relative to the control, identifies the development of irAE due to immune checkpoint therapy as being less likely. In some embodiments of this aspect, the methods further include recommending, prescribing, or administering the immune checkpoint therapy if the development of irAE due to immune checkpoint therapy is determined to be less likely or administering an anti-cancer therapy other than the immune checkpoint therapy if the development of irAE due to immune checkpoint therapy is determined to be more likely.

In an aspect of the invention, methods of selecting a subject having cancer for treatment with an immune checkpoint inhibitor (ICI) include a) measuring the amount of at least one germline biomarker listed in Table 1 in the subject sample; b) comparing said amount of the at least one germline biomarker listed in Table 1 to a control; and c) selecting the subject for treatment with an ICI if the at least one germline biomarker listed in Table 1 is absent or has a significantly decreased amount in the subject sample relative to the control.

In an aspect of the invention, methods of selecting a subject having cancer for treatment with an anti-cancer therapy other than the immune checkpoint therapy include a) measuring the amount of at least one germline biomarker listed in Table 1 in the subject sample; b) comparing said amount of the at least one germline biomarker listed in Table 1 to a control; and c) selecting the subject for treatment with an anti-cancer therapy other than the immune checkpoint therapy if the at least one germline biomarker listed in Table 1 is present or has a significantly increased amount in the subject sample relative to the control.

In an aspect of the invention, methods of treating a subject having cancer with an immune checkpoint inhibitor (ICI) include a) selecting a subject for treatment with an ICI, wherein the subject has been determined to have at least one germline biomarker listed in Table 1 that is absent or has a significantly decreased amount in the subject sample relative to a control; and b) administering an ICI to the subject.

In an aspect of the invention, methods of treating a subject having cancer with an anti-cancer therapy other than the immune checkpoint therapy include a) selecting a subject for treatment with an anti-cancer therapy other than the immune checkpoint therapy, wherein the subject has been determined to have at least one germline biomarker listed in Table 1 that is present or has a significantly increased amount in the subject sample relative to a control; and b) administering an anti-cancer therapy other than the immune checkpoint therapy to the subject.

Numerous embodiments are further provided that can be applied to any aspect of the present invention and/or combined with any other embodiment described herein. For example, in some embodiments, the anti-cancer therapy is selected from the group consisting of targeted therapy, chemotherapy, radiation therapy, and/or hormonal therapy. In some embodiments, the control is determined from a cancerous or non-cancerous sample from one or more members of the same species to which the subject belongs. In some embodiments, the control is determined from a cancerous or non-cancerous sample representative of said one or more members who received immune checkpoint therapy but either did not develop irAE or developed irAE to an extent not exceeding a permissible level. In some embodiments, the amount of the at least one germline biomarker listed in Table 1 is detected using a whole exome sequencing method. In some embodiments, the amount of the at least one germline biomarker listed in Table 1 is detected by imputing it from its amount in a non-germline sample (e.g., tumor sample). In some embodiments, the germline biomarker is a germline SNP. In some embodiments, the subject sample and/or the control is selected from the group consisting of *ex vivo* and *in vivo* samples. In some embodiments, the subject sample and/or the control is a portion of a single sample or pooled samples obtained from the subject. In some embodiments, the subject sample and/or the control has not been contacted with any anti-cancer treatment or inhibitor of an immune checkpoint. In some embodiments, the subject has not been administered any anti-cancer treatment or inhibitor of an immune checkpoint. In some embodiments, the subject sample is selected from the group consisting of serum, whole blood, plasma, urine, cells, cell lines, and biopsies. In some embodiments, the amount of the at least one germline biomarker listed in Table 1 is detected using a reagent which specifically binds with the germline biomarker. In some embodiments, the reagent has a label group attached thereto, optionally wherein the label group comprising: a radioisotope, a fluorescent compound, an enzyme, or an enzyme co-factor. In some embodiments, the at least one germline biomarker listed in Table 1 is assessed by liquid chromatography tandem mass spectrometry (LC-MS), HPLC, and/or mass spectrometry. In some embodiments, the step of detecting further comprises purifying and/or concentrating the at least one germline biomarker listed in Table 1. In some embodiments, the at least one germline biomarker listed in Table 1 is somatic CNA amplification TMPRSS2, somatic CNA amplification NSD1, somatic CNA amplification UIMC1, somatic CNA amplification FGFR4, somatic CNA amplification RUNX1, somatic CNA amplification TDG, CNA deletion WAS, CNA deletion ARAF, somatic SNV mutation in ARID1A, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), germline risk score for blood cell counts, germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs). In some embodiments, the immune checkpoint therapy comprises an inhibitor of at least one selected from the group consisting of PD-1, PD-L1, PD-L2, TIM-3, LAG-3, CTLA-4, and combinations thereof. In some embodiments, the inhibitor comprises at least one antibody selected from the group consisting of anti-PD-1 antibodies, anti-CTLA4 antibodies, anti-PD-L1 antibodies, anti-PD-L2 antibodies, and combinations thereof. In some embodiments, the immune checkpoint therapy comprises an anti-PD-1 antibody. In some embodiments, the cancer is selected from the group consisting of melanoma, bladder cancer, and renal cell cancer (RCC). In some embodiments, the subject is a mammal (*e.g.,* an animal model of cancer, a human, a rodent).

### Brief Description of Figures

The patent of application file contains at least one drawing executed in color. Copies of this patent or patent application publication with color drawing(s) will be provided by the office upon request and payment of the necessary fee.

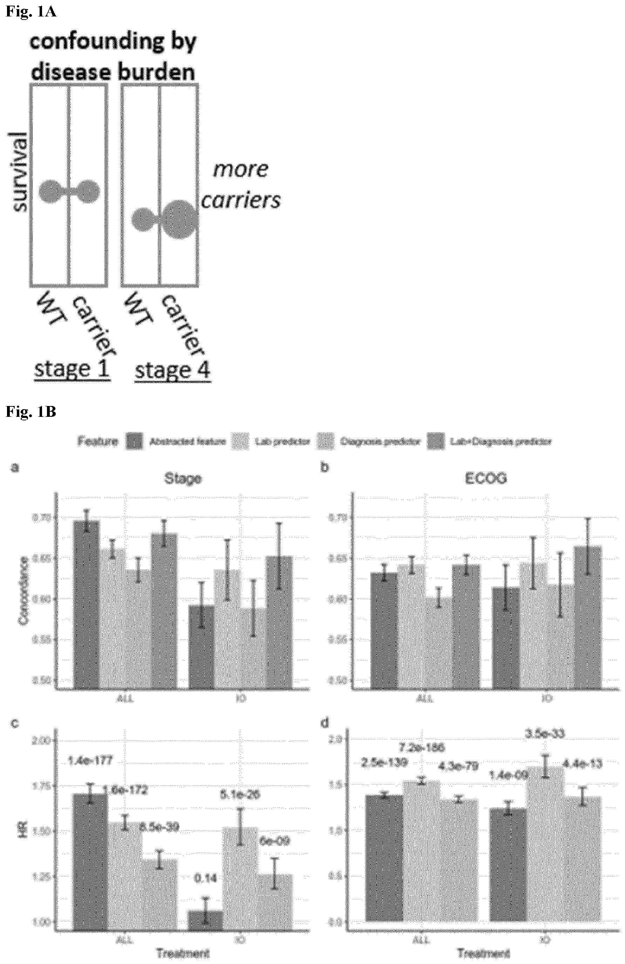

**FIG. 1A** and FIG. 1B show possible confounding in retrospective data. (1A) Disease burden can cause associations between overall survival (OS) and covariates. (1B) Adjusting for disease burden by imputing stage and ECOG status at treatment start for all patients from lab values and diagnosis codes in the full cohort of 60,000 patients. The predictors are highly significant and prognostic.

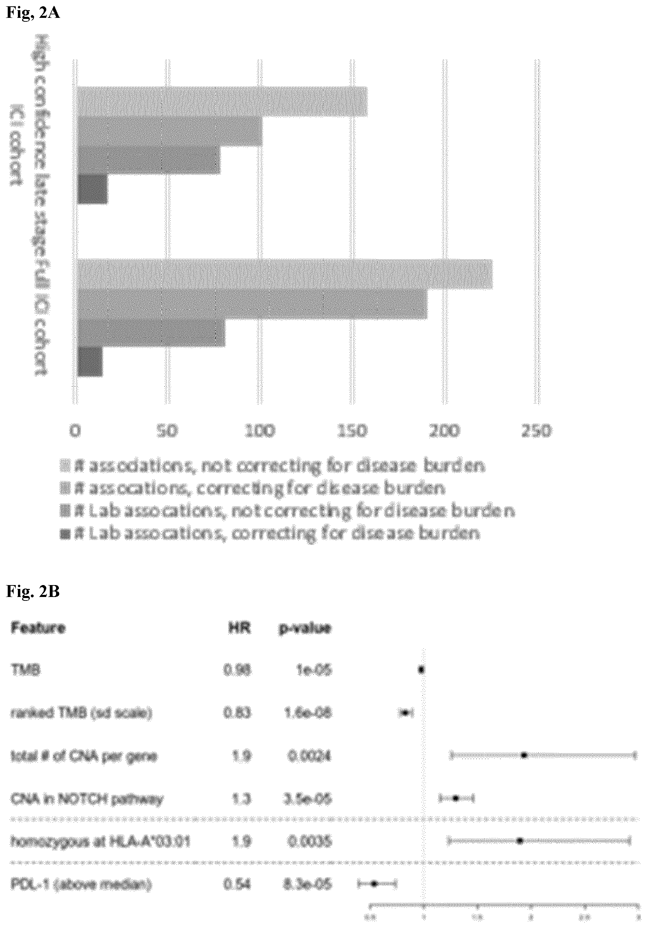

**FIG. 2A** and **FIG. 2B** show that controlling for disease burden explains many false associations with OS. (Fig. 2A) Controlling for disease burden, the number of associations with OS decreases, especially for lab values, which are easily influenced by disease burden. (Fig. 2B) Validating associations with OS.

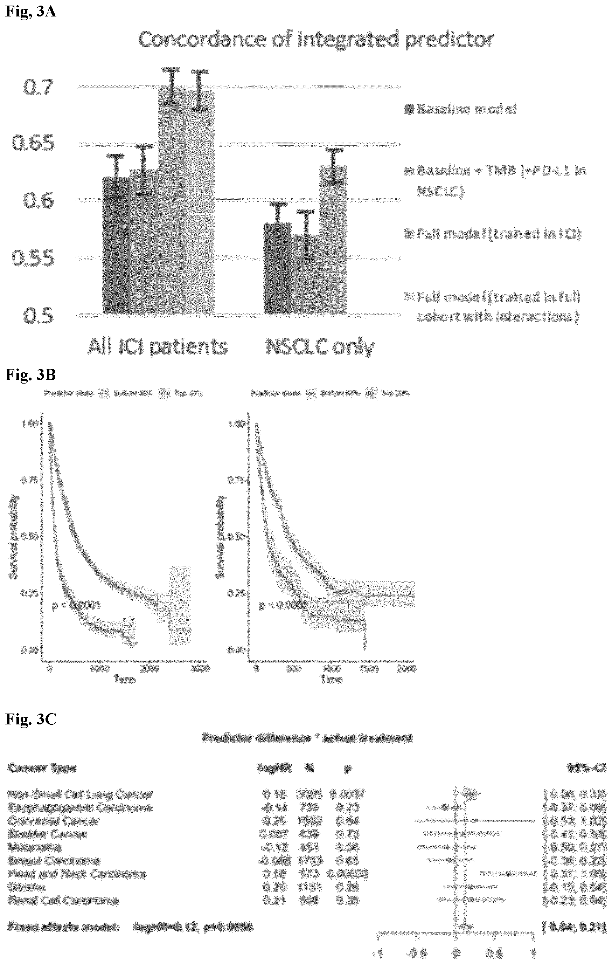

FIG. 3A - FIG. 3C show predictive integrated interaction model. (Fig. 3A) All available features are integrated into a pheno-marker predicting OS. Baseline model: age, gender, treatment, start year, line of treatment Additional features: germline, somatic, labs, imputed ECOG/stage. (Figs. 3B and 3C) Time dependent interaction analysis: being on ICIs significantly worse if predicted to be worse (Fig. 3B: predictor is prognostic; Fig. 3C: predictor is predictive).

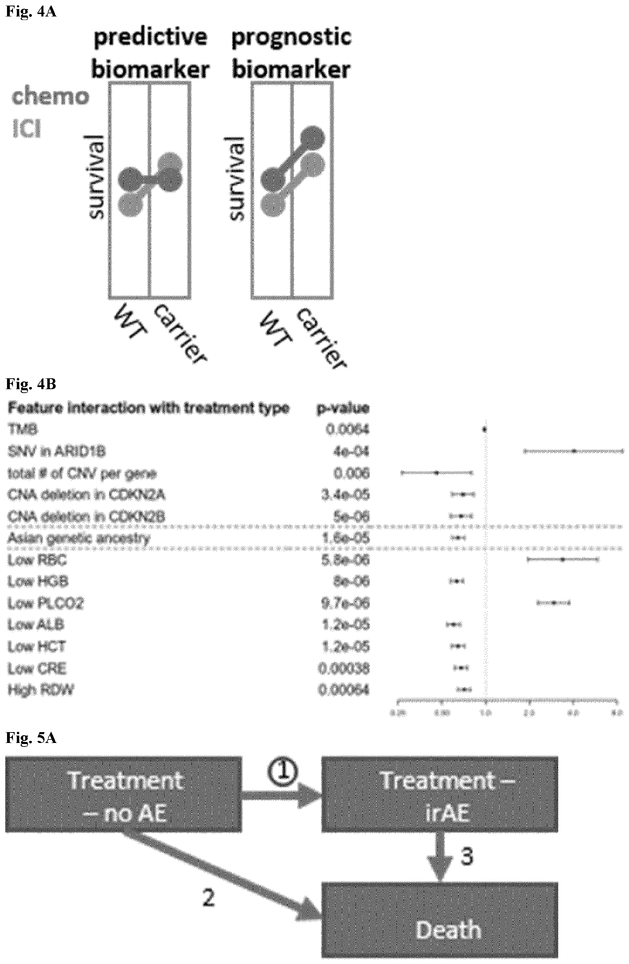

**FIG. 4A** and **FIG. 4B** show ICI specific biomarkers for OS. (Fig. 4A) To find associations that are not just prognostic but predictive, an interaction survival analysis is performed with treatment type. (Fig. 4B) Many significant ICI-specific biomarkers, some previously found: TMB (Samstein 2019), SNV in PBRM1 (RCC, Miao, Science 2018), CDKN2A (association with PDL-1 levels in NSCLC, Zhang, Journal of Clinical Oncology 2018).

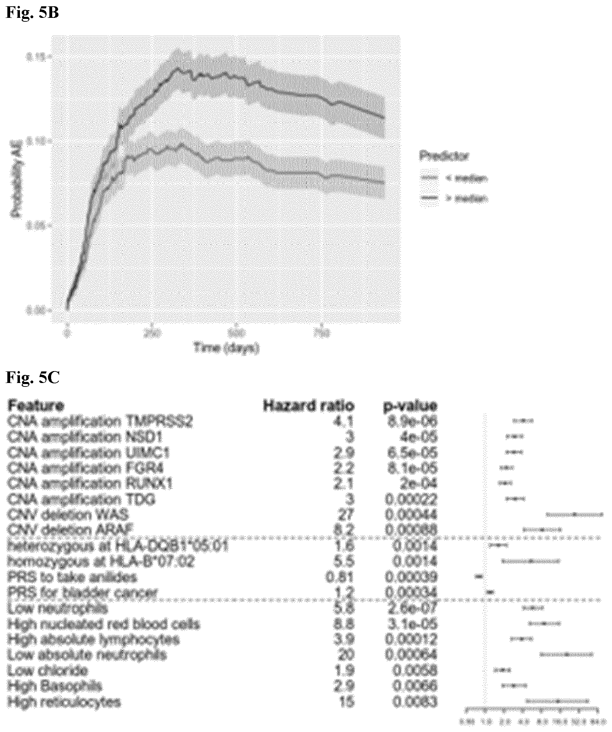

FIG. 5A - FIG. 5C show associations with irAEs. (Fig. 5A) To model irAEs, an additional state in survival model is shown. This model takes care of competing hazards, which are important for irAE prediction. Many (Fig. 5C) associations with irAEs are found and are able to build a preliminary predictor, which can identify significantly different incidence rate of irAEs (Fig. 5B).

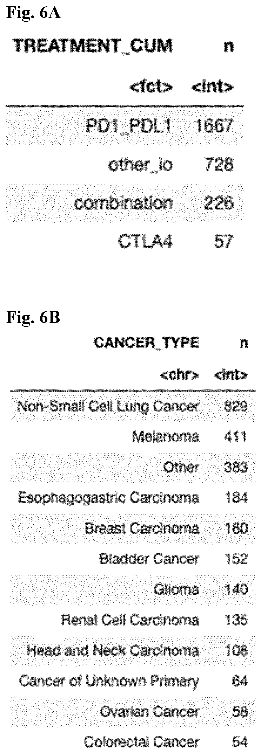

**FIG. 6A** and **FIG. 6B** show data related to 2,600 pan-cancer patients on immune checkpoint inhibitors. Tumor panel sequencing used to identify germline features. Adverse Events (AE) quantified from electronic medical records / EPIC (Fig. 6A: Treatment; Fig. 6B: Cancer).

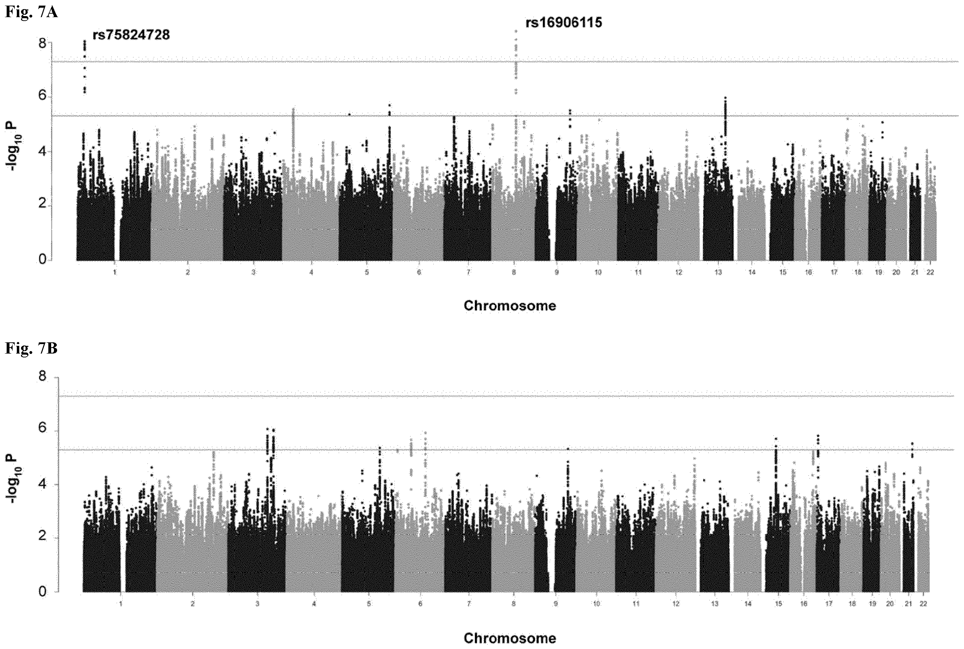

**FIG. 7A** and **Fig. 7B** show results of an exemplary genome-wide association study to determine whether any single germline mutation associated with AE. The study tests each common germline variant (~1M in total) for association with an AE during treatment, and modeling competing risk of death. The results include multiple significant associations, and an ability to construct a "risk score."

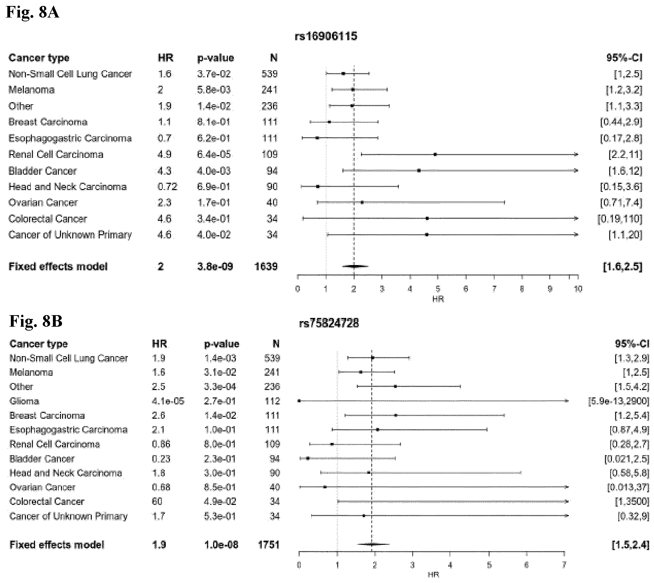

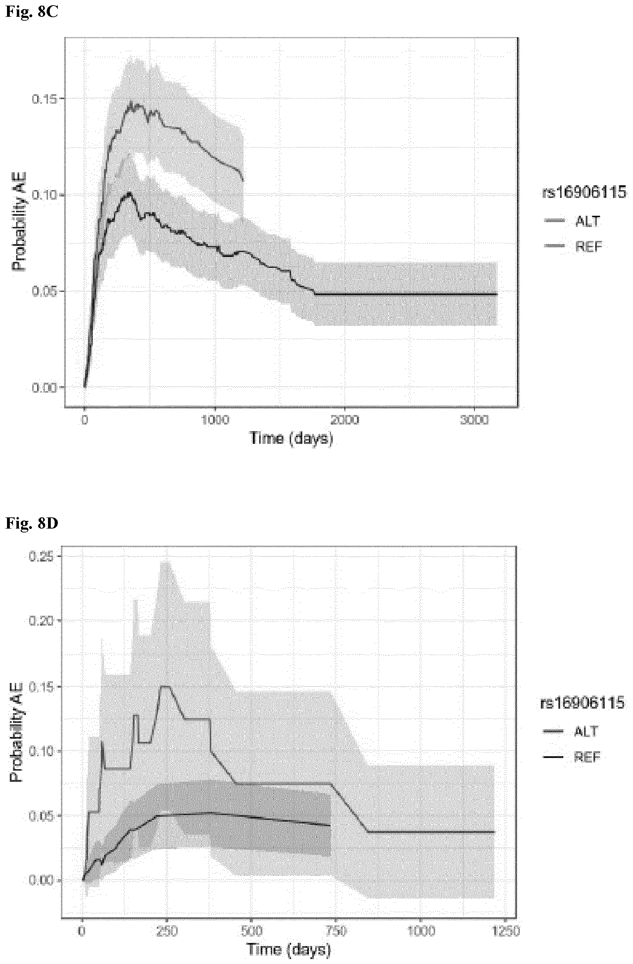

FIG. 8A - FIG. 8D show sample results for IL7 SNP association (Figs. 8A and 8B: graph; Figs. 8C and 8D: table).

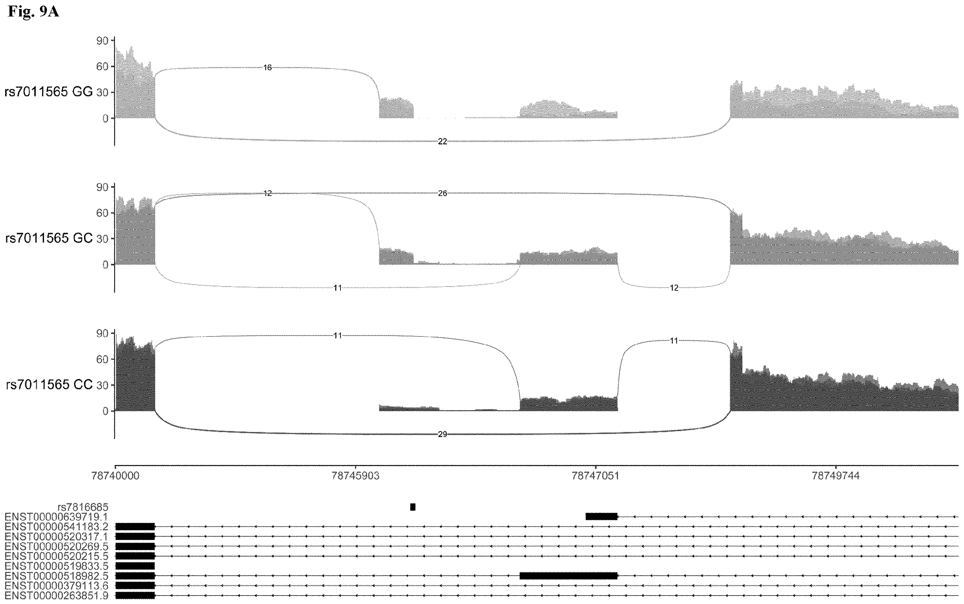

FIG. 9A - FIG. 9D show the effect of rs7816685 on IL7 splicing.

### Detailed Description of the Invention

It has been determined herein that certain germline mutations can be specific biomarkers for predicted development of irAE in a wide variety of cancers afflicting patients who have received anti-immune checkpoint-based therapy (e.g., PD-1 inhibitors). Accordingly, the present invention relates, in part, to methods for stratifying patients and predicting development of irAE in a subject receiving immune checkpoint therapy based upon a determination and analysis of amounts of such biomarkers, compared to a control. In addition, such analyses can be used to provide useful anti-immune checkpoint treatment regimens (*e.g*., based on the predicted likelihood of development if irAE).

The findings are based, in part, on the development of a computational algorithm to infer germline (inherited) genetic variation directly from tumor sequencing data, which data is widely available and routinely collected in the clinic. Specific germline variants were identified that are associated with an increased incidence of irAEs for patients on ICIs in real world data from the Dana-Farber Profile project. As germline variation is fixed at birth, these predictors are actionable as early as cancer diagnosis and allow for stratification of patients for irAE risk.

### I. Definitions

The articles "a" and "an" are used herein to refer to one or to more than one (i.e. to at least one) of the grammatical object of the article. By way of example, "an element" means one element or more than one element.

The term "altered amount" or "altered level" refers to increased or decreased level or concentration (measurable in either absolute or relatively modulated ways) of a biomarker compound, *e.g.,* increased or decreased level or concentration in a cancer sample, as compared to the level or concentration of the biomarker compound in a control sample. The term "altered amount" of a biomarker also includes an increased or decreased level or concentration of a biomarker compound in a sample, *e.g.,* a cancer sample, as compared to the corresponding level or concentration in a normal, control sample. The altered amount or level is greater or less than the standard error of the assay employed to assess the level or concentration, and is preferably at least 20%, 30%, 40%, 50%, 60%, 70%, 80%, 90%, 100%, 150%, 200%, 300%, 350%, 400%, 500%, 600%, 700%, 800%, 900%, 1000% or more times the level or concentration of the biomarker in a control sample (*e.g.,* sample from a healthy subjects not having the associated disease) and preferably, the average level or concentration of the biomarker in several control samples.

The amount of a biomarker in a subject is "significantly" higher or lower than the normal amount of the biomarker, if the amount of the biomarker is greater or less, respectively, than the normal level by an amount greater than the standard error of the assay employed to assess amount, and preferably at least 20%, 30%, 40%, 50%, 60%, 70%, 80%, 90%, 100%, 150%, 200%, 300%, 350%, 400%, 500%, 600%, 700%, 800%, 900%, 1000% or than that amount. As described further below, such "significance" can be assessed from any desired or known point of comparison, such as a particular post-treatment versus pretreatment biomarker measurement measurement, comparison to a control measurement, comparison to a normal measurement, comparison to a group of samples or subjects, and the like. In some embodiments, a "significantly" higher or lower amount can be measured as a ratio, such as 1-, 1.1-, 1.2-, 1.3-, 1.4-, 1.5-, 1.6-, 1.7-. 1.8-, 1.9-, 2.0-, 2.1-, 2.2-, 2.3-, 2.4-, 2.5-, 2.6-, 2.7-, 2.8-, 2.9-, 3.0-, 3.1-, 3.2-, 3.3-, 3.4-, 3.5-, 3.6-, 3.7-, 3.8-, 3.9-, 4.0-, 4.1-, 4.2-, 4.3-, 4.4-, 4.5-, 4.6-, 4.7-, 4.8-, 4.9-, 5.0-, 5.1-, 5.2-, 5.3-, 5.4-, 5.5-, 5.6-, 5.7-, 5.8-, 5.9-, 6.0-, 6.1-, 6.2-, 6.3-, 6.4-, 6.5-, 6.6-, 6.7-, 6.8-, 6.9-, 7.0-, 7.1-, 7.2-, 7.3-, 7.4-, 7.5-, 7.6-, 7.7-, 7.8-, 7.9-, 8.0-, 8.1-, 8.2-, 8.3-, 8.4-, 8.5-, 8.6-, 8.7-, 8.8-, 8.9-, 9.0-, 9.1-, 9.2-, 9.3-, 9.4-, 9.5-, 9.6-, 9.7-, 9.8-, 9.9-, 10-, 11-, 12-, 13-, 14-, 15-, 16-, 17-, 18-, 19-, 20-fold change, or more, or any range in between, inclusive (*e.g*., 1.2-fold to 11-fold change). In some embodiments, the fold-change can be linear or logarithmic, such as a fold change measured as log2 or log10 units. In other embodiments, the measurement is an absolute amount (*e.g*., 100 nM, 200 nM, 300 nM, 500 nM, 1000 nM, and the like). In still other embodiments, the measurement can be made over different points of time and/or interventions, such as at baseline, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, and the like. Such "significance" can also be applied to any other measured parameter described herein, such as for expression, inhibition, cytotoxicity, cell growth, and the like.

The term "allelic variant of a polymorphic region of gene" or "allelic variant", used interchangeably herein, refers to an alternative form of a gene having one of several possible nucleotide sequences found in that region of the gene in the population. As used herein, allelic variant is meant to encompass functional allelic variants, non-functional allelic variants, SNPs, mutations and polymorphisms.

The term "single nucleotide polymorphism" (SNP) refers to a polymorphic site occupied by a single nucleotide, which is the site of variation between allelic sequences. The site is usually preceded by and followed by highly conserved sequences of the allele (*e.g.,* sequences that vary in less than 1/100 or 1/1000 members of a population). A SNP usually arises due to substitution of one nucleotide for another at the polymorphic site. SNPs can also arise from a deletion of a nucleotide or an insertion of a nucleotide relative to a reference allele. Typically the polymorphic site is occupied by a base other than the reference base. For example, where the reference allele contains the base "T" (thymidine) at the polymorphic site, the altered allele can contain a "C" (cytidine), "G" (guanine), or "A" (adenine) at the polymorphic site. SNP's may occur in protein-coding nucleic acid sequences, in which case they may give rise to a defective or otherwise variant protein, or genetic disease. Such a SNP may alter the coding sequence of the gene and therefore specify another amino acid (a "missense" SNP) or a SNP may introduce a stop codon (a "nonsense" SNP). When a SNP does not alter the amino acid sequence of a protein, the SNP is called "silent." SNP's may also occur in noncoding regions of the nucleotide sequence. This may result in defective protein expression, *e.g*., as a result of alternative spicing, or it may have no effect on the function of the protein.

Unless otherwise specified here within, the terms "antibody" and "antibodies" broadly encompass naturally-occurring forms of antibodies (*e.g.* IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical moiety conjugated to an antibody.

The term "antibody" as used herein also includes an "antigen-binding portion" of an antibody (or simply "antibody portion"). The term "antigen-binding portion", as used herein, refers to one or more fragments of an antibody that retain the ability to specifically bind to an antigen (*e.g.,* a biomarker polypeptide or fragment thereof). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent polypeptides (known as single chain Fv (scFv); see *e.g.,* Bird et al. (1988) Science 242:423-426; and Huston et al. (1988) Proc. Natl. Acad. Sci. USA 85:5879-5883; and Osbourn et al. 1998, Nature Biotechnology 16: 778). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG polypeptides or other isotypes. VH and VL can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R. J., et al. (1994) Structure 2:1121-1123).

Still further, an antibody or antigen-binding portion thereof may be part of larger immunoadhesion polypeptides, formed by covalent or noncovalent association of the antibody or antibody portion with one or more other proteins or peptides. Examples of such immunoadhesion polypeptides include use of the streptavidin core region to make a tetrameric scFv polypeptide (Kipriyanov, S.M., et al. (1995) Human Antibodies and Hybridomas 6:93-101) and use of a cysteine residue, biomarker peptide and a C-terminal polyhistidine tag to make bivalent and biotinylated scFv polypeptides (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion polypeptides can be obtained using standard recombinant DNA techniques, as described herein.

Antibodies may be polyclonal or monoclonal; xenogeneic, allogeneic, or syngeneic; or modified forms thereof (*e.g.* humanized, chimeric, etc.). Antibodies may also be fully human. Preferably, antibodies of the present invention bind specifically or substantially specifically to a biomarker polypeptide or fragment thereof. The terms "monoclonal antibodies" and "monoclonal antibody composition", as used herein, refer to a population of antibody polypeptides that contain only one species of an antigen binding site capable of immunoreacting with a particular epitope of an antigen, whereas the term "polyclonal antibodies" and "polyclonal antibody composition" refer to a population of antibody polypeptides that contain multiple species of antigen binding sites capable of interacting with a particular antigen. A monoclonal antibody composition typically displays a single binding affinity for a particular antigen with which it immunoreacts.

Antibodies may also be "humanized", which is intended to include antibodies made by a non-human cell having variable and constant regions which have been altered to more closely resemble antibodies that would be made by a human cell. For example, by altering the non-human antibody amino acid sequence to incorporate amino acids found in human germline immunoglobulin sequences. The humanized antibodies of the present invention may include amino acid residues not encoded by human germline immunoglobulin sequences (*e.g.,* mutations introduced by random or site-specific mutagenesis *in vitro* or by somatic mutation *in vivo*), for example in the CDRs. The term "humanized antibody", as used herein, also includes antibodies in which CDR sequences derived from the germline of another mammalian species, such as a mouse, have been grafted onto human framework sequences.

The term "assigned score" refers to the numerical value designated for each of the biomarkers after being measured in a patient sample. The assigned score correlates to the absence, presence or inferred amount of the biomarker in the sample. The assigned score can be generated manually (*e.g.,* by visual inspection) or with the aid of instrumentation for image acquisition and analysis. In certain embodiments, the assigned score is determined by a qualitative assessment, for example, detection of a fluorescent readout on a graded scale, or quantitative assessment. In one embodiment, an "aggregate score," which refers to the combination of assigned scores from a plurality of measured biomarkers, is determined. In one embodiment the aggregate score is a summation of assigned scores. In another embodiment, combination of assigned scores involves performing mathematical operations on the assigned scores before combining them into an aggregate score. In certain, embodiments, the aggregate score is also referred to herein as the "predictive score."

The term "biomarker" refers to a measurable entity of the present invention that has been determined to be predictive of the development of immune-related adverse events due to immune checkpoint therapy of a cancer. Biomarkers can include, without limitation, germline variations (*e.g.,* allelic variants, SNPs), including those shown in Table 1 below, the Examples, and the Figures.

### Table 1

Somatic CNA amplification TMPRSS2
   (Transmembrane serine protease 2 gene present on human chromosome 21q22.3 located at NC_000021.9 (41464305..41508158, complement) of human genome reference GRCh38.p13 and having Gene ID 7113 available on the world wide web at ncbi.nlm.nih.gov/gene/7113) (including, without limitation, shallow amplifications (gain of one copy), deep amplifications (gain of more than one copy), as well as a quantitative estimate of the number of copies lost or gained)
Somatic CNA amplification NSD1
   (Nuclear receptor binding SET Domain Protein 1 on human chromosome 5q35.3 of human genome GRCh38.p13 and having Gene ID 64324 available on the world wide web at ncbi.nlm.nih.gov/gene/64324) (including, without limitation, shallow amplifications (gain of one copy), deep amplifications (gain of more than one copy), as well as a quantitative estimate of the number of copies lost or gained)
Somatic CNA amplification UIMC1
   (ubiquitin interaction motif containing 1 on human chromosome 5q35.2 of human genome GRCh38.p13 and having Gene ID 51720 available on the world wide web at ncbi.nlm.nih.gov/gene/51720) (including, without limitation, shallow amplifications (gain of one copy), deep amplifications (gain of more than one copy), as well as a quantitative estimate of the number of copies lost or gained)
Somatic CNA amplification FGFR4
   (fibroblast growth factor receptor 4 on human chromosome 5q35.2 of human genome GRCh38.p13 and having Gene ID 2264 available on the world wide web at ncbi.nlm.nih.gov/gene/2264) (including, without limitation, shallow amplifications (gain of one copy), deep amplifications (gain of more than one copy), as well as a quantitative estimate of the number of copies lost or gained)
Somatic CNA amplification RUNX1
   (RUNX family transcription factor 1 on human chromosome 21q22.1 of human genome GRCh38.p13 and having Gene ID 861 available on the world wide web at ncbi.nlm.nih.gov/gene/861) (including, without limitation, shallow amplifications (gain of one copy), deep amplifications (gain of more than one copy), as well as a quantitative estimate of the number of copies lost or gained)
Somatic CNA amplification TDG
   (thymine DNA glycosylase on human chromosome 12q23.3 of human genome GRCh38.p13 and having Gene ID 6996 available on the world wide web at ncbi.nlm.nih.gov/gene/6996) (including, without limitation, shallow amplifications (gain of one copy), deep amplifications (gain of more than one copy), as well as a quantitative estimate of the number of copies lost or gained)
CNA deletion WAS
   (WASP actin nucleation promoting factor on human chromosome Xp 11.23 of human genome GRCh38.p13 and having Gene ID 7454 available on the world wide web at ncbi.nlm.nih.gov/gene/7454) (including, without limitation, shallow deletions (loss of one copy), deep deletions (loss of more than one copy), as well as a quantitative estimate of the number of copies lost or gained)
CNA deletion ARAF
   (A-Raf proto-oncogene, serine/threonine kinase on human chromosome Xp11.3 of human genome GRCh38.p13 and having Gene ID 369 available on the world wide web at ncbi.nlm.nih.gov/gene/369) (including, without limitation, shallow deletions (loss of one copy), deep deletions (loss of more than one copy), as well as a quantitative estimate of the number of copies lost or gained)
Somatic SNV mutation in ARID1A
   (AT-rich interaction domain 1A on human chromosome 1p36.11 of human genome GRCh38.p13 and having Gene ID 8289 available on the world wide web at ncbi.nlm.nih.gov/gene/8289) (including, without limitation, truncating mutations, nonsynonymous mutations, splice variants, as well as a quantitative estimate of the number of somatic coding mutations in the gene)
SNV mutation in ARID1B
   (AT-rich interaction domain 1B on human chromosome 6q25.3 of human genome GRCh38.p13 and having Gene ID 57492 available on the world wide web at ncbi.nlm.nih.gov/gene/57492) (including, without limitation, truncating mutations, nonsynonymous mutations, splice variants, as well as a quantitative estimate of the number of somatic coding mutations in the gene)
SNV mutation in PIK3R1
   (phosphoinositide-3-kinase regulatory subunit 1 on human chromosome 5q13.1 of human genome GRCh38.p13 and having Gene ID 5295 available on the world wide web at ncbi.nlm.nih.gov/gene/5295) (including, without limitation, truncating mutations, nonsynonymous mutations, splice variants, as well as a quantitative estimate of the number of somatic coding mutations in the gene)
SNV mutation in APC
   (APC regulator of WNT signaling pathway on human chromosome 5q22.2 of human genome GRCh38.p13 and having Gene ID 324 available on the world wide web at ncbi.nlm.nih.gov/gene/324) (including, without limitation, truncating mutations, nonsynonymous mutations, splice variants, as well as a quantitative estimate of the number of somatic coding mutations in the gene)
SNV mutation in SETD2
   (SET domain containing 2, histone lysine methyltransferase on human chromosome 3p21.31 of human genome GRCh38.p13 and having Gene ID 29072 available on the world wide web at ncbi.nlm.nih.gov/gene/29072) (including, without limitation, truncating mutations, nonsynonymous mutations, splice variants, as well as a quantitative estimate of the number of somatic coding mutations in the gene)
SNV mutation in B2M
   (beta-2-microglobulin on human chromosome 15q21.1 of human genome GRCh38.p13 and having Gene ID 567 available on the world wide web at ncbi.nlm.nih.gov/gene/567) (including, without limitation, truncating mutations, nonsynonymous mutations, splice variants, as well as a quantitative estimate of the number of somatic coding mutations in the gene)
SNV mutation in BCOR
   (BCL6 corepressor on human chromosome Xp11.4 of human genome GRCh38.p13 and having Gene ID 54880 available on the world wide web at ncbi.nlm.nih.gov/gene/54880) (including, without limitation, truncating mutations, nonsynonymous mutations, splice variants, as well as a quantitative estimate of the number of somatic coding mutations in the gene)
SNV mutation in CASP8
   (caspase 8 on human chromosome 2q33.1 of human genome GRCh38.p13 and having Gene ID 841 available on the world wide web at ncbi.nlm.nih.gov/gene/841) (including, without limitation, truncating mutations, nonsynonymous mutations, splice variants, as well as a quantitative estimate of the number of somatic coding mutations in the gene)
SNV mutation in KDM6A
   (lysine demethylase 6A on human chromosome Xp11.3 of human genome GRCh38.p13 and having Gene ID 7403 available on the world wide web at ncbi.nlm.nih.gov/gene/7403) (including, without limitation, truncating mutations, nonsynonymous mutations, splice variants, as well as a quantitative estimate of the number of somatic coding mutations in the gene)
SNV mutation in MSH6
   (mutS homolog 6 on human chromosome 2p16.3 of human genome GRCh38.p13 and having Gene ID 2956 available on the world wide web at ncbi.nlm.nih.gov/gene/2956) (including, without limitation, truncating mutations, nonsynonymous mutations, splice variants, as well as a quantitative estimate of the number of somatic coding mutations in the gene)
SNV mutation in ROS1
   (ROS proto-oncogene 1, receptor tyrosine kinase on human chromosome 6q22.1 of human genome GRCh38.p13 and having Gene ID 6098 available on the world wide web at ncbi.nlm.nih.gov/gene/6098) (including, without limitation, truncating mutations, nonsynonymous mutations, splice variants, as well as a quantitative estimate of the number of somatic coding mutations in the gene)
Germline risk score for bladder cancer, for medication use (including anti-inflammatory or immunosuppressant medications), and for blood cell counts (including lymphocyte and neutrophil counts). Germline risk scores may be computed, as demonstrated in the working examples, using all common polymorphisms associated with the focal trait (e.g., bladder cancer or medication use), weighted by their association with the focal trait including if desired, without limitation, weights computed from the estimated causal (e.g., fine-mapped) effect on the focal trait, weights adjusted for linkage disequilibrium correlations among variants, and weights pruned based on linkage disequilibrium correlations among variants.
Germline variant near the IL7 gene or influencing IL7 splicing (SNP rsid rs7816685, or SNP rsid rs16906115, or other correlated SNPs with an R^2 value of >0.5)
   (rs7816685 is a T>C substitution at chromosome 8:78746671 (human genome reference GRCh38.p13) near gene IL7 having canonical SPDI
   NC_000008.11:g.78746671T>C available on the world wide web at ncbi.nlm.nih.gov/snp/rs7816685)
   (rs16906115 is a G>A substitution at chromosome 8:78800763 (human genome reference GRCh38.p13) near gene IL7 having canonical SPDI
   NC_000008.11:g.78800763G>A available on the world wide web at ncbi.nlm.nih.gov/snp/rs16906115)
Germline variant near the IL22RA1 gene (SNP rsid rs75824728 and correlated SNPs with an R^2 value of >0.5)
   (rs75824728 is a G>A substitution at chromosome 1:24138136 (human genome reference GRCh38.p13) near gene IL22RA1 having canonical SPDI
   NC_0 0 0 0 0 1. 11: g. 2413 8 13 6 G>A available on the world wide web at ncbi.nlm.nih.gov/snp/rs75824728)
* Representative, non-limiting SNPs correlated with SNPs described herein are presented in Table 2 and sub-tables thereof as follows:

**Table 2**

| Table 2A: SNPs correlated with SNP rs7816685 and/or SNP rs 16906115 |
|---|
| rs7816685 is a T>C substitution at chromosome 8:78746671 (human genome reference GRCh38.p13) near gene IL7 having canonical SPDI NC_000008.11:g.78746671T>C available on the world wide web at ncbi.nlm.nih.gov/snp/rs7816685 |
| rs16906115 is a G>A substitution at chromosome 8:78800763 (human genome reference GRCh38.p13) near gene IL7 having canonical SPDI NC_000008.11:g.78800763G>A available on the world wide web at ncbi.nlm.nih.gov/snp/rs16906115 |
| rs7011565 is a C>A,G substitution at chromosome 8:78645002 (human genome reference GRCh38.p13) near gene LOC105375911 having canonical SPDI NC_000008.11:g.78645002C>A,NC_000008.11:g.78645002C>G available on the world wide web at ncbi.nlm.nih.gov/snp/rs7011565 |
| rs73249933 is a T>C substitution at chromosome 8:78714040 (human genome reference GRCh38.p13) near gene IL7/ZC2HC1A having canonical SPDI NC_000008.11:g.78714040T>C available on the world wide web at ncbi.nlm.nih.gov/snp/rs73249933 |
| rs74197776 is a C>A substitution at chromosome 8:78743947 (human genome reference GRCh38.p13) near gene IL7 having canonical SPDINC_000008.11:g.78743947C>A available on the world wide web at ncbi.nlm.nih.gov/snp/rs74197776 |
| rs16906062 is a T>A,G substitution at chromosome 8:78746947 (human genome reference GRCh38.p13) near gene IL7 having canonical SPDI NC_000008.11:g.78746947T>A,NC_000008.11:g.78746947T>G available on the world wide web at ncbi.nlm.nih.gov/snp/rs16906062 |
| rs34096063 is a T>C,G substitution at chromosome 8:78753072 (human genome reference GRCh38.p13) near gene IL7 having canonical SPDI NC_000008.11:g.78753072T>C,NC_000008.11:g.78753072T>G available on the world wide web at ncbi.nlm.nih.gov/snp/rs34096063 |
| rs2369536 is a C>G,T substitution at chromosome 8:78753094 (human genome reference GRCh38.p13) near gene IL7 having canonical SPDI NC_000008.11:g.78753094C>G,NC_000008.11:g.78753094C>T available on the world wide web at ncbi.nlm.nih.gov/snp/rs2369536 |
| rs4739137 is a C>T substitution at chromosome 8:78761612 (human genome reference GRCh38.p13) near gene IL7 having canonical SPDINC_000008.11:g.78761612C>T available on the world wide web at ncbi.nlm.nih.gov/snp/rs4739137 |
| rs73251644 is a C>T substitution at chromosome 8:78782229 (human genome reference GRCh38.p13) near gene IL7 having canonical SPDINC_000008.11:g.78782229C>T available on the world wide web at ncbi.nlm.nih.gov/snp/rs73251644 |

| Table 2B: SNPs correlated with SNP rs75824728 |
|---|
| rs75824728 is a G>A substitution at chromosome 1:24138136 (human genome reference GRCh38.p13) near gene IL22RA1 having canonical SPDI NC_000001.11:g.24138136G>A available on the world wide web at ncbi.nlm.nih.gov/snp/rs75824728 |
| rs75492368 is a C>T substitution at chromosome 1:24125230 (human genome reference GRCh38.p13) near gene IL22RA1 having canonical SPDI NC_000001.11:g.24125230C>T available on the world wide web at ncbi.nlm.nih.gov/snp/rs75492368 |
| rs79895649 is a C>T substitution at chromosome 1:24128412 (human genome reference GRCh38.p13) near gene IL22RA1 having canonical SPDI NC_000001.11:g.24128412C>T available on the world wide web at ncbi.nlm.nih.gov/snp/rs79895649 |
| rs75345424 is a C>G,T substitution at chromosome 1:24131788 (human genome reference GRCh38.p13) near gene IL22RA1 having canonical SPDI NC_000001.11:g.24131788C>G,NC_000001.11:g.24131788C>T available on the world wide web at ncbi.nlm.nih.gov/snp/rs75345424 |
| rs144697207 is a C>G,T substitution at chromosome 1:24132424 (human genome reference GRCh38.p13) near gene IL22RA1 having canonical SPDI NC_000001.11:g.24132424C>G,NC_000001.11:g.24132424C>T available on the world wide web at ncbi.nlm.nih.gov/snp/rs144697207 |
| rs78086250 is a C>A substitution at chromosome 1:24136869 (human genome reference GRCh38.p13) near gene IL22RA1 having canonical SPDI NC_000001.11:g.24136869C>A available on the world wide web at ncbi.nlm.nih.gov/snp/rs78086250 |
| rs7418238 is a A>G substitution at chromosome 1:24136927 (human genome reference GRCh38.p13) near gene IL22RA1 having canonical SPDI NC_000001.11:g.24136927A>G available on the world wide web at ncbi.nlm.nih.gov/snp/rs7418238 |
| rs75032062 is a C>A substitution at chromosome 1:24138102 (human genome reference GRCh38.p13) near gene IL22RA1 having canonical SPDI NC_000001.11:g.24138102C>A available on the world wide web at ncbi.nlm.nih.gov/snp/rs75032062 |
| rs115203657 is a G>A,T substitution at chromosome 1:24145089 (human genome reference GRCh38.p13) near gene IL22RA1 having canonical SPDI NC_000001.11:g.24145089G>A,NC_000001.11:g.24145089G>T available on the world wide web at ncbi.nlm.nih.gov/snp/rs115203657 |
| rs77279721 is a C>T substitution at chromosome 1:24145574 (human genome reference GRCh38.p13) having canonical SPDINC_000001.11:g.24145574C>T available on the world wide web at ncbi.nlm.nih.gov/snp/rs77279721 |
| rs6424157 is a C>G,T substitution at chromosome 1:24147386 (human genome reference GRCh38.p13) having canonical SPDI NC_000001.11:g.24147386C>G,NC_000001.11:g.241473 86C>T available on the world wide web at ncbi.nlm.nih.gov/snp/rs6424157 |

| |
|---|
| * For any SNP correlation described in Table 1, Table 2 or any sub-table thereof, and/or in the specification, an R^2 value may be greater than or equal to 0.5, 0.55, 0.6, 0.65, 0.7, 0.75, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, or greater, or any range in between inclusive, such as 0.95 to 0.99. |

A "blocking" antibody or an antibody "antagonist" is one which inhibits or reduces at least one biological activity of the antigen(s) it binds. In certain embodiments, the blocking antibodies or antagonist antibodies or fragments thereof described herein substantially or completely inhibit a given biological activity of the antigen(s).

The term "body fluid" refers to fluids that are excreted or secreted from the body as well as fluids that are normally not (*e.g*., amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit, and the like).

The terms "cancer" or "tumor" or "hyperproliferative" refer to the presence of cells possessing characteristics typical of cancer-causing cells, such as uncontrolled proliferation, immortality, metastatic potential, rapid growth and proliferation rate, and certain characteristic morphological features. In some embodiments, such cells exhibit such characteristics in part or in full due to the expression and activity of immune checkpoint proteins, such as PD-1, PD-L1, PD-L2, TIM, LAG, and/or CTLA-4. Cancer cells are often in the form of a tumor, but such cells may exist alone within an animal, or may be a non-tumorigenic cancer cell, such as a leukemia cell. As used herein, the term "cancer" includes premalignant as well as malignant cancers. Cancers include, but are not limited to, B cell cancer, *e.g.,* multiple myeloma, Waldenström's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematologic tissues, and the like. Other non-limiting examples of types of cancers applicable to the methods encompassed by the present invention include human sarcomas and carcinomas, *e.g.,* fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, cancers are epithlelial in nature and include but are not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (e.g., serous ovarian carcinoma), or breast carcinoma. The epithelial cancers may be characterized in various other ways including, but not limited to, serous, endometrioid, mucinous, clear cell, Brenner, or undifferentiated. In some embodiments, the cancer or tumor is melanoma and/or renal cell cancer (RCC).

The terms "conjoint therapy" and "combination therapy," as used herein, refer to the administration of two or more therapeutic substances, *e.g.,* combinations of anti-immune checkpoint therapies, multiple inhibitors of an immune checkpoint of interest, combinations of immune checkpoint therapy with an inhibitor of PD-1, PD-L1, PD-L2, TIM, LAG, CTLA-4, and the like), and combinations thereof. The different agents comprising the combination therapy may be administered concomitant with, prior to, or following the administration of one or more therapeutic agents.

The term "control" refers to any reference standard suitable to provide a comparison to the expression products in the test sample. In one embodiment, the control comprises obtaining a "control sample" from which expression product levels are detected and compared to the expression product levels from the test sample. Such a control sample may comprise any suitable sample, including but not limited to a sample from a control cancer patient (can be stored sample or previous sample measurement) with a known outcome; normal tissue or cells isolated from a subject, such as a normal patient or the cancer patient, cultured primary cells/tissues isolated from a subject such as a normal subject or the cancer patient, adjacent normal cells/tissues obtained from the same organ or body location of the cancer patient, a tissue or cell sample isolated from a normal subject, or a primary cells/tissues obtained from a depository. In another preferred embodiment, the control may comprise a reference standard expression product level from any suitable source, including but not limited to housekeeping genes, an expression product level range from normal tissue (or other previously analyzed control sample), a previously determined expression product level range within a test sample from a group of patients, or a set of patients with a certain outcome (for example, survival for one, two, three, four years, etc.) or receiving a certain treatment (for example, standard of care cancer therapy). It will be understood by those of skill in the art that such control samples and reference standard expression product levels can be used in combination as controls in the methods of the present invention. In one embodiment, the control may comprise normal or non-cancerous cell/tissue sample. In another preferred embodiment, the control may comprise an expression level for a set of patients, such as a set of cancer patients, or for a set of cancer patients receiving a certain treatment, or for a set of patients with one outcome versus another outcome. In the former case, the specific expression product level of each patient can be assigned to a percentile level of expression, or expressed as either higher or lower than the mean or average of the reference standard expression level. In another preferred embodiment, the control may comprise normal cells, cells from patients treated with combination chemotherapy, and cells from patients having benign cancer. In another embodiment, the control may also comprise a measured value for example, average level of expression of a particular gene in a population compared to the level of expression of a housekeeping gene in the same population. Such a population may comprise normal subjects, cancer patients who have not undergone any treatment (*i.e.,* treatment naive), cancer patients undergoing standard of care therapy, or patients having benign cancer. In another preferred embodiment, the control comprises a ratio transformation of expression product levels, including but not limited to determining a ratio of expression product levels of two genes in the test sample and comparing it to any suitable ratio of the same two genes in a reference standard; determining expression product levels of the two or more genes in the test sample and determining a difference in expression product levels in any suitable control; and determining expression product levels of the two or more genes in the test sample, normalizing their expression to expression of housekeeping genes in the test sample, and comparing to any suitable control. In particularly preferred embodiments, the control comprises a control sample which is of the same lineage and/or type as the test sample. In another embodiment, the control may comprise expression product levels grouped as percentiles within or based on a set of patient samples, such as all patients with cancer. In one embodiment a control expression product level is established wherein higher or lower levels of expression product relative to, for instance, a particular percentile, are used as the basis for predicting outcome. In another preferred embodiment, a control expression product level is established using expression product levels from cancer control patients with a known outcome, and the expression product levels from the test sample are compared to the control expression product level as the basis for predicting outcome. As demonstrated by the data below, the methods of the present invention are not limited to use of a specific cut-point in comparing the level of expression product in the test sample to the control.

The "copy number" of a biomarker nucleic acid refers to the number of DNA sequences in a cell (*e.g.,* germline and/or somatic) encoding a particular gene product. Generally, for a given gene, a mammal has two copies of each gene. The copy number can be increased, however, by gene amplification or duplication, or reduced by deletion. For example, germline copy number changes include changes at one or more genomic loci, wherein said one or more genomic loci are not accounted for by the number of copies in the normal complement of germline copies in a control (*e.g.,* the normal copy number in germline DNA for the same species as that from which the specific germline DNA and corresponding copy number were determined). Somatic copy number changes include changes at one or more genomic loci, wherein said one or more genomic loci are not accounted for by the number of copies in germline DNA of a control (*e.g.,* copy number in germline DNA for the same subject as that from which the somatic DNA and corresponding copy number were determined).

The "normal" copy number (*e.g.,* germline and/or somatic) of a biomarker nucleic acid or "normal" level of expression of a biomarker nucleic acid, protein, or metabolite is the activity/level of expression or copy number in a biological sample, *e.g.,* a sample containing tissue, whole blood, serum, plasma, buccal scrape, saliva, cerebrospinal fluid, urine, stool, and bone marrow, from a subject, *e.g.,* a human, not afflicted with cancer, or from a corresponding non-cancerous tissue in the same subject who has cancer.

As used herein, the term "costimulate" with reference to activated immune cells includes the ability of a costimulatory molecule to provide a second, non-activating receptor mediated signal (a "costimulatory signal") that induces proliferation or effector function. For example, a costimulatory signal can result in cytokine secretion, *e.g.,* in a T cell that has received a T cell-receptor-mediated signal. Immune cells that have received a cell-receptor mediated signal, *e.g.,* via an activating receptor are referred to herein as "activated immune cells."

The term "determining a suitable treatment regimen for the subject" is taken to mean the determination of a treatment regimen (*i*.*e*., a single therapy or a combination of different therapies that are used for the prevention and/or treatment of the cancer in the subject) for a subject that is started, modified and/or ended based or essentially based or at least partially based on the results of the analysis according to the present invention. One example is determining whether to provide targeted therapy against a cancer to provide immunotherapy that generally increases immune responses against the cancer (*e.g.,* using immune checkpoint therapy when it is determined that irAE development is less likely). Another example is starting an adjuvant therapy after surgery whose purpose is to decrease the risk of recurrence, another would be to modify the dosage of a particular chemotherapy. The determination can, in addition to the results of the analysis according to the present invention, be based on personal characteristics of the subject to be treated. In most cases, the actual determination of the suitable treatment regimen for the subject will be performed by the attending physician or doctor.

The term "diagnosing cancer" includes the use of the methods, systems, and code of the present invention to determine the presence or absence of a cancer or subtype thereof in an individual. The term also includes methods, systems, and code for assessing the level of disease activity in an individual.

A molecule is "fixed" or "affixed" to a substrate if it is covalently or non-covalently associated with the substrate such that the substrate can be rinsed with a fluid (*e.g.* standard saline citrate, pH 7.4) without a substantial fraction of the molecule dissociating from the substrate.

The term "expression signature" or "signature" refers to a group of two or more coordinately expressed biomarkers. For example, the genes, proteins, metabolites, and the like making up this signature may be expressed in a specific cell lineage, stage of differentiation, or during a particular biological response. The biomarkers can reflect biological aspects of the tumors in which they are expressed, such as the cell of origin of the cancer, the nature of the non-malignant cells in the biopsy, and the oncogenic mechanisms responsible for the cancer. Expression data and gene expression levels can be stored on computer readable media, *e.g.,* the computer readable medium used in conjunction with a microarray or chip reading device. Such expression data can be manipulated to generate expression signatures.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue. By way of example, a region having the nucleotide sequence 5'-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

The term "immune cell" refers to cells that play a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes.

The term "immune checkpoint" refers to a group of molecules on the cell surface of CD4+ and/or CD8+ T cells that fine-tune immune responses by down-modulating or inhibiting an anti-tumor immune response. Immune checkpoint proteins are well known in the art and include, without limitation, CTLA-4, PD-1, VISTA, B7-H2, B7-H3, PD-L1, B7-H4, B7-H6, 2B4, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, and A2aR (see, for example, WO 2012/177624). The term further encompasses biologically active protein fragment, as well as nucleic acids encoding full-length immune checkpoint proteins and biologically active protein fragments thereof. In some embodiment, the term further encompasses any fragment according to homology descriptions provided herein.

"Immune checkpoint therapy" or "immune checkpoint inhibitors (ICIs)" refer to the use of agents that inhibit immune checkpoint nucleic acids and/or proteins. Inhibition of one or more immune checkpoints can block or otherwise neutralize inhibitory signaling to thereby upregulate an immune response in order to more efficaciously treat cancer. Exemplary agents useful for inhibiting immune checkpoints include antibodies, small molecules, peptides, peptidomimetics, natural ligands, and derivatives of natural ligands, that can either bind and/or inactivate or inhibit immune checkpoint proteins, or fragments thereof; as well as RNA interference, antisense, nucleic acid aptamers, etc. that can downregulate the expression and/or activity of immune checkpoint nucleic acids, or fragments thereof. Exemplary agents for upregulating an immune response include antibodies against one or more immune checkpoint proteins block the interaction between the proteins and its natural receptor(s); a non-activating form of one or more immune checkpoint proteins (*e.g.,* a dominant negative polypeptide); small molecules or peptides that block the interaction between one or more immune checkpoint proteins and its natural receptor(s); fusion proteins (*e.g.* the extracellular portion of an immune checkpoint inhibition protein fused to the Fc portion of an antibody or immunoglobulin) that bind to its natural receptor(s); nucleic acid molecules that block immune checkpoint nucleic acid transcription or translation; and the like. Such agents can directly block the interaction between the one or more immune checkpoints and its natural receptor(s) (*e.g.,* antibodies) to prevent inhibitory signaling and upregulate an immune response. Alternatively, agents can indirectly block the interaction between one or more immune checkpoint proteins and its natural receptor(s) to prevent inhibitory signaling and upregulate an immune response. For example, a soluble version of an immune checkpoint protein ligand such as a stabilized extracellular domain can binding to its receptor to indirectly reduce the effective concentration of the receptor to bind to an appropriate ligand. In one embodiment, anti-PD-1 antibodies, anti-PD-L1 anti-PD-L2 antibodies, and anti-CTLA-4 antibodies, either alone or in combination, are used to inhibit immune checkpoints.

In some examples, the at least one anti-PD-1 agent is selected from the group consisting of cemiplimab (REGN2810), nivolumab (BMS-936558, MDX-1106, ONO-4538), pembrolizumab (MK-3475, SCH 900475), SHR1210, sintilimab (IBI308), spartalizumab (PDR001), tislelizumab (BGB-A317), pidilizumab, BCD-100, toripalimab (JS001), PF-06801591, AB122, AK105, AMG 404, BCD-100, BI 754091, F520, HLX10, HX008, JTX-4014, LZM009, MEDI0680, MGA012, Sym021, TSR-042, PSB205, MGD019, MGD013, AK104, XmAb20717, RO7121661, and CX-188. In some examples, the list of anti-PD-L1 agents is selected from the group consisting of atezolizumab (MPDL3280A, RG7446, RO5541267), durvalumab (MEDI4736, MEDI-4736), avelumab (MSB0010718C), FS118, BCD-135, BGB-A333, CBT-502, CK-301, CS1001, FAZ053, HLX20, KN035, MDX-1105, MSB2311, SHR-1316, TG-1501, ZKAB001, INBRX-105, MCLA-145, KN046, M7824, and LY3415244.

"Ipilimumab" is a representative example of an immune checkpoint therapy. Ipilimumab (previously MDX-010; Medarex Inc., marketed by Bristol-Myers Squibb as YERVOY^{™}) is a fully human anti-human CTLA-4 monoclonal antibody that blocks the binding of CTLA-4 to CD80 and CD86 expressed on antigen presenting cells, thereby, blocking the negative down-regulation of the immune responses elicited by the interaction of these molecules (see, for example, WO 2013/169971, U.S. Pat. Publ. 2002/0086014, and U.S. Pat. Publ. 2003/0086930.

"Nivolumab" is another representative example of an immune checkpoint therapy. Nivolumab (discovered by Medarex, developed by Medarex and Ono Pharmaceutical, and marketed by Bristol-Myers Squibb and Ono as Opdivo^{®}) is a human IgG4 anti-PD-1 monoclonal antibody and works as a checkpoint inhibitor, blocking signals that would have prevented activated T cells from attacking the cancer, thus allowing the immune system to clear the cancer. It is used as a first line treatment for inoperable or metastatic melanoma in combination with ipilimumab if the cancer does not have a mutation in BRAF, as a second-line treatment following treatment with ipilimumab and if the cancer has a mutation in BRAF, with a BRAF inhibitor (Johnson et al. (2015) Ther Adv Med Oncol. 7:97-106), as a second-line treatment for squamous non-small cell lung cancer (Sundar et al. (2015) Ther Adv Med Oncol. 7:85-96), and as a second-line treatment for renal cell carcinoma. FDA has approved nivolumab for primary or metastatic urothelial carcinoma, the most common form of bladder cancer. It can be prescribed for locally advanced or metastatic form of the condition that experience disease progression during or following platinum-containing chemotherapy or have progression within 12 months of neoadjuvant or adjuvant treatment with platinum-containing chemotherapy.

The term "immune response" includes T cell mediated and/or B cell mediated immune responses. Exemplary immune responses include T cell responses, *e.g.,* cytokine production and cellular cytotoxicity. In addition, the term immune response includes immune responses that are indirectly effected by T cell activation, *e.g.,* antibody production (humoral responses) and activation of cytokine responsive cells, *e.g.,* macrophages.

The term "immune-related adverse event" or "irAE" includes side effects that occur due to (i.e., while undergoing, or after undergoing) immune-checkpoint therapy. These side effects are caused by an immune response to the therapy, and can range from mild to severe.

The term "immunotherapeutic agent" can include any molecule, peptide, antibody or other agent which can stimulate a host immune system to generate an immune response to a tumor or cancer in the subject. Various immunotherapeutic agents are useful in the compositions and methods described herein.

The term "inhibit" includes the decrease, limitation, or blockage, of, for example a particular action, function, or interaction. In some embodiments, cancer is "inhibited" if at least one symptom of the cancer is alleviated, terminated, slowed, or prevented. As used herein, cancer is also "inhibited" if recurrence or metastasis of the cancer is reduced, slowed, delayed, or prevented.

The term "interaction", when referring to an interaction between two molecules, refers to the physical contact (*e.g.,* binding) of the molecules with one another. Generally, such an interaction results in an activity (which produces a biological effect) of one or both of said molecules.

An "isolated protein" refers to a protein that is substantially free of other proteins, cellular material, separation medium, and culture medium when isolated from cells or produced by recombinant DNA techniques, or chemical precursors or other chemicals when chemically synthesized. An "isolated" or "purified" protein or biologically active portion thereof is substantially free of cellular material or other contaminating proteins from the cell or tissue source from which the antibody, polypeptide, peptide or fusion protein is derived, or substantially free from chemical precursors or other chemicals when chemically synthesized.

The language "substantially free of cellular material" includes preparations of a biomarker polypeptide or fragment thereof, in which the protein is separated from cellular components of the cells from which it is isolated or recombinantly produced. In one embodiment, the language "substantially free of cellular material" includes preparations of a biomarker protein or fragment thereof, having less than about 30% (by dry weight) of non-biomarker protein (also referred to herein as a "contaminating protein"), more preferably less than about 20% of non-biomarker protein, still more preferably less than about 10% of non-biomarker protein, and most preferably less than about 5% non-biomarker protein. When antibody, polypeptide, peptide or fusion protein or fragment thereof, *e.g.,* a biologically active fragment thereof, is recombinantly produced, it is also preferably substantially free of culture medium, *i.e*., culture medium represents less than about 20%, more preferably less than about 10%, and most preferably less than about 5% of the volume of the protein preparation.

A "kit" is any manufacture (*e.g.* a package or container) comprising at least one reagent, *e.g.* a probe or small molecule, for specifically detecting and/or affecting the expression of a marker of the present invention. The kit may be promoted, distributed, or sold as a unit for performing the methods of the present invention. The kit may comprise one or more reagents necessary to express a composition useful in the methods of the present invention. In certain embodiments, the kit may further comprise a reference standard, *e.g.,* a nucleic acid encoding a protein that does not affect or regulate signaling pathways controlling cell growth, division, migration, survival or apoptosis. One skilled in the art can envision many such control proteins, including, but not limited to, common molecular tags (*e.g.,* green fluorescent protein and beta-galactosidase), proteins not classified in any of pathway encompassing cell growth, division, migration, survival or apoptosis by GeneOntology reference, or ubiquitous housekeeping proteins. Reagents in the kit may be provided in individual containers or as mixtures of two or more reagents in a single container. In addition, instructional materials which describe the use of the compositions within the kit can be included.

The term "neoadjuvant therapy" refers to a treatment given before the primary treatment. Examples of neoadjuvant therapy can include chemotherapy, radiation therapy, and hormone therapy. For example, in treating breast cancer, neoadjuvant therapy can allows patients with large breast cancer to undergo breast-conserving surgery.

The "normal" level of expression of a biomarker is the level of expression of the biomarker in cells of a subject, *e.g.,* a human patient, not afflicted with a cancer. An "overexpression" or "significantly higher level or amount" of a biomarker refers to a level or amount in a test sample that is greater than the standard error of the assay employed to assess expression, and is preferably at least 10%, and more preferably 1.2, 1.3, 1.4, 1.5, 1.6, 1.7, 1.8, 1.9, 2.0, 2.1, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher than the level or amount of the biomarker in a control sample (*e.g.,* sample from a healthy subject not having the biomarker associated disease) and preferably, the average level or amount of the biomarker in several control samples. For example, in one embodiment, a "significantly lower level or amount" of a biomarker refers to a level or amount in a test sample that is at least 10%, and more preferably 1.2, 1.3, 1.4, 1.5, 1.6, 1.7, 1.8, 1.9, 2.0, 2.1, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more lower than the level or amount of the biomarker in a control sample (*e.g.,* sample from a healthy subject not having the biomarker associated disease or from a sample from a subject prior to or during therapy) and preferably, the average level or amount of the biomarker in several control samples. In the same manner, a "significantly higher level or amount" of a biomarker can be defined but in the opposite direction.

The term "pre-determined" biomarker amount and/or activity measurement(s) may be a biomarker amount and/or activity measurement(s) used to, by way of example only, evaluate a subject that may be selected for a particular treatment, evaluate the likelihood of developing an irAE when an anti-immune checkpoint inhibitor therapy is to be used, and/or evaluate the disease state. A pre-determined biomarker amount and/or activity measurement(s) may be determined in populations of patients with or without cancer. The pre-determined biomarker amount and/or activity measurement(s) can be a single number, equally applicable to every patient, or the pre-determined biomarker amount and/or activity measurement(s) can vary according to specific subpopulations of patients. Age, weight, height, and other factors of a subject may affect the pre-determined biomarker amount and/or activity measurement(s) of the individual. Furthermore, the pre-determined biomarker amount and/or activity can be determined for each subject individually. In one embodiment, the amounts determined and/or compared in a method described herein are based on absolute measurements. In another embodiment, the amounts determined and/or compared in a method described herein are based on relative measurements, such as ratios (*e.g.,* serum biomarker normalized to the expression of a housekeeping or otherwise generally constant biomarker). The pre-determined biomarker amount and/or activity measurement(s) can be any suitable standard. For example, the pre-determined biomarker amount and/or activity measurement(s) can be obtained from the same or a different human for whom a patient selection is being assessed. In one embodiment, the pre-determined biomarker amount and/or activity measurement(s) can be obtained from a previous assessment of the same patient. In such a manner, the progress of the selection of the patient can be monitored over time. In addition, the control can be obtained from an assessment of another human or multiple humans, *e.g.,* selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group.

The term "predictive" includes the use of a biomarker compound status, e.g., over- or under- activity, emergence, expression, growth, remission, recurrence or resistance of tumors before, during or after therapy, for determining the likelihood of development of irAE due to anti-immune checkpoint treatment (*e.g.,* therapeutic antibodies against CTLA-4, PD-1, PD-L1, and the like). Such predictive use of the biomarker may be confirmed by, *e.g.,* (1) increased or decreased copy number (*e.g.,* by FISH, FISH plus SKY, single-molecule sequencing, *e.g.,* as described in the art at least at J. Biotechnol., 86:289-301, or qPCR), overexpression or underexpression of a biomarker nucleic acid (*e.g.,* by ISH, Northern Blot, or qPCR), increased or decreased biomarker protein (*e.g.,* by IHC), or increased or decreased activity, e.g., in more than about 5%, 6%, 7%, 8%, 9%, 10%, 11%, 12%, 13%, 14%, 15%, 20%, 25%, 30%, 40%, 50%, 60%, 70%, 80%, 90%, 95%, 100%, or more of assayed human cancers types or cancer samples; (2) its absolute or relatively modulated presence or absence in a biological sample, *e.g.,* a sample containing tissue, whole blood, serum, plasma, buccal scrape, saliva, cerebrospinal fluid, urine, stool, or bone marrow, from a subject, *e.g.* a human, afflicted with cancer; (3) its absolute or relatively modulated presence or absence in clinical subset of patients with cancer (*e.g.,* those responding to a particular immune checkpoint therapy or those developing resistance thereto).

The term "pre-malignant lesions" as described herein refers to a lesion that, while not cancerous, has potential for becoming cancerous. It also includes the term "pre-malignant disorders" or "potentially malignant disorders." In particular this refers to a benign, morphologically and/or histologically altered tissue that has a greater than normal risk of malignant transformation, and a disease or a patient's habit that does not necessarily alter the clinical appearance of local tissue but is associated with a greater than normal risk of precancerous lesion or cancer development in that tissue (leukoplakia, erythroplakia, erytroleukoplakia lichen planus (lichenoid reaction) and any lesion or an area which histological examination showed atypia of cells or dysplasia.

The terms "prevent," "preventing," "prevention," "prophylactic treatment," and the like refer to reducing the probability of developing a disease, disorder, or condition in a subject, who does not have, but is at risk of or susceptible to developing a disease, disorder, or condition.

The term "probe" refers to any molecule which is capable of selectively binding to a specifically intended target molecule, for example, a biomarker compound. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic molecules.

The term "prognosis" includes a prediction of the probable course and outcome of cancer or the likelihood of recovery from the disease. In some embodiments, the use of statistical algorithms provides a prognosis of cancer in an individual. For example, the prognosis can be surgery, development of a clinical subtype of cancer *(e.g.,* solid tumors, such as lung cancer, melanoma, and renal cell carcinoma), development of one or more clinical factors, development of intestinal cancer, or recovery from the disease.

The term "response to immune checkpoint therapy" or "response to therapy" relates to any response of the hyperproliferative disorder (*e.g.,* cancer) to a therapy, such as an immune checkpoint therapy like immune checkpoint therapy, preferably to a change in tumor mass and/or volume after initiation of neoadjuvant or adjuvant chemotherapy. Hyperproliferative disorder response may be assessed, for example for efficacy or in a neoadjuvant or adjuvant situation, where the size of a tumor after systemic intervention can be compared to the initial size and dimensions as measured by CT, PET, mammogram, ultrasound or palpation. Responses may also be assessed by caliper measurement or pathological examination of the tumor after biopsy or surgical resection. Response may be recorded in a quantitative fashion like percentage change in tumor volume or in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of hyperproliferative disorder response may be done early after the onset of neoadjuvant or adjuvant therapy, *e.g.,* after a few hours, days, weeks or preferably after a few months. A typical endpoint for response assessment is upon termination of neoadjuvant chemotherapy or upon surgical removal of residual tumor cells and/or the tumor bed. This is typically three months after initiation of neoadjuvant therapy. In some embodiments, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments, the CBR for a particular cancer therapeutic regimen is at least 25%, 30%, 35%, 40%, 45%, 50%, 55%, 60%, 65%, 70%, 75%, 80%, 85%, or more. Additional criteria for evaluating the response to cancer therapies are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.,* time of diagnosis or start of treatment) and end point (*e.g.,* death, recurrence or metastasis). In addition, criteria for efficacy of treatment can be expanded to include response to chemotherapy, probability of survival, probability of metastasis within a given time period, and probability of tumor recurrence. For example, in order to determine appropriate threshold values, a particular cancer therapeutic regimen can be administered to a population of subjects and the outcome can be correlated to biomarker measurements that were determined prior to administration of any cancer therapy. The outcome measurement may be pathologic response to therapy given in the neoadjuvant setting. Alternatively, outcome measures, such as overall survival and disease-free survival can be monitored over a period of time for subjects following cancer therapy for whom biomarker measurement values are known. In certain embodiments, the doses administered are standard doses known in the art for cancer therapeutic agents. The period of time for which subjects are monitored can vary. For example, subjects may be monitored for at least 1, 2, 3, 4, 5, 6, 7, 8, 9, 10, 11, 12, 13, 14, 15, 16, 17, 18, 19, 20 weeks or longer, such as 2, 4, 6, 8, 10, 12, 14, 16, 18, 20, 25, 30, 35, 40, 45, 50, 55, or 60 months. Biomarker measurement threshold values that correlate to outcome of a cancer therapy can be determined using well-known methods in the art, such as those described in the Examples section.

The term "resistance" refers to an acquired or natural resistance of a cancer sample or a mammal to a cancer therapy ( *i.e*., being nonresponsive to or having reduced or limited response to the therapeutic treatment), such as having a reduced response to a therapeutic treatment by 25% or more, for example, 30%, 40%, 50%, 60%, 70%, 80%, or more, to 2-fold, 3-fold, 4-fold, 5-fold, 10-fold, 15-fold, 20-fold or more. The reduction in response can be measured by comparing with the same cancer sample or mammal before the resistance is acquired, or by comparing with a different cancer sample or a mammal who is known to have no resistance to the therapeutic treatment. A typical acquired resistance to chemotherapy is called "multidrug resistance." The multidrug resistance can be mediated by P-glycoprotein or can be mediated by other mechanisms, or it can occur when a mammal is infected with a multi-drug-resistant microorganism or a combination of microorganisms. The determination of resistance to a therapeutic treatment is routine in the art and within the skill of an ordinarily skilled clinician, for example, can be measured by cell proliferative assays and cell death assays as described herein as "sensitizing." In some embodiments, the term "reverses resistance" means that the use of a second agent in combination with a primary cancer therapy (*e.g.,* chemotherapeutic or radiation therapy) is able to produce a significant decrease in tumor volume at a level of statistical significance (*e.g.,* p<0.05) when compared to tumor volume of untreated tumor in the circumstance where the primary cancer therapy (*e.g.,* chemotherapeutic or radiation therapy) alone is unable to produce a statistically significant decrease in tumor volume compared to tumor volume of untreated tumor. This generally applies to tumor volume measurements made at a time when the untreated tumor is growing log rhythmically.

The terms "response" or "responsiveness" refers to an anti-cancer response, *e.g*. in the sense of reduction of tumor size or inhibiting tumor growth. The terms can also refer to an improved prognosis, for example, as reflected by an increased time to recurrence, which is the period to first recurrence censoring for second primary cancer as a first event or death without evidence of recurrence, or an increased overall survival, which is the period from treatment to death from any cause. To respond or to have a response means there is a beneficial endpoint attained when exposed to a stimulus. Alternatively, a negative or detrimental symptom is minimized, mitigated or attenuated on exposure to a stimulus. It will be appreciated that evaluating the likelihood that a tumor or subject will exhibit a favorable response is equivalent to evaluating the likelihood that the tumor or subject will not exhibit favorable response (*i.e.,* will exhibit a lack of response or be non-responsive).

An "RNA interfering agent" as used herein, is defined as any agent which interferes with or inhibits expression of a target biomarker gene by RNA interference (RNAi). Such RNA interfering agents include, but are not limited to, nucleic acid molecules including RNA molecules which are homologous to the target biomarker gene of the present invention, or a fragment thereof, short interfering RNA (siRNA), and small molecules which interfere with or inhibit expression of a target biomarker nucleic acid by RNA interference (RNAi).

"RNA interference (RNAi)" is an evolutionally conserved process whereby the expression or introduction of RNA of a sequence that is identical or highly similar to a target biomarker nucleic acid results in the sequence specific degradation or specific post-transcriptional gene silencing (PTGS) of messenger RNA (mRNA) transcribed from that targeted gene (*see* Coburn, G. and Cullen, B. (2002) J. of Virology 76(18):9225), thereby inhibiting expression of the target biomarker nucleic acid. In one embodiment, the RNA is double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex that recognizes and cleaves target mRNAs. RNAi can also be initiated by introducing nucleic acid molecules, *e.g.,* synthetic siRNAs or RNA interfering agents, to inhibit or silence the expression of target biomarker nucleic acids. As used herein, "inhibition of target biomarker nucleic acid expression" or "inhibition of marker gene expression" includes any decrease in expression or protein activity or level of the target biomarker nucleic acid or protein encoded by the target biomarker nucleic acid. The decrease may be of at least 30%, 40%, 50%, 60%, 70%, 80%, 90%, 95% or 99% or more as compared to the expression of a target biomarker nucleic acid or the activity or level of the protein encoded by a target biomarker nucleic acid which has not been targeted by an RNA interfering agent.

The term "sample" used for detecting or determining the presence or level of at least one biomarker is typically whole blood, plasma, serum, saliva, urine, stool (*e.g.,* feces), tears, and any other bodily fluid (*e.g.,* as described above under the definition of "body fluids"), or a tissue sample (*e.g.,* biopsy) such as a small intestine, colon sample, or surgical resection tissue. In certain instances, the method of the present invention further comprises obtaining the sample from the individual prior to detecting or determining the presence or level of at least one marker in the sample.

The term "sensitize" means to alter cancer cells or tumor cells in a way that allows for more effective treatment of the associated cancer with a cancer therapy (*e.g.,* anti-immune checkpoint, chemotherapeutic, and/or radiation therapy). In some embodiments, normal cells are not affected to an extent that causes the normal cells to be unduly injured by the immune checkpoint therapy. An increased sensitivity or a reduced sensitivity to a therapeutic treatment is measured according to a known method in the art for the particular treatment and methods described herein below, including, but not limited to, cell proliferative assays (Tanigawa N, Kern D H, Kikasa Y, Morton D L, Cancer Res 1982; 42: 2159-2164), cell death assays (Weisenthal L M, Shoemaker RH, Marsden J A, Dill P L, Baker J A, Moran E M, Cancer Res 1984; 94: 161-173; Weisenthal L M, Lippman M E, Cancer Treat Rep 1985; 69: 615-632; Weisenthal L M, In: Kaspers G J L, Pieters R, Twentyman P R, Weisenthal L M, Veerman A J P, eds. Drug Resistance in Leukemia and Lymphoma. Langhome, P A: Harwood Academic Publishers, 1993: 415-432; Weisenthal L M, Contrib Gynecol Obstet 1994; 19: 82-90). The sensitivity or resistance may also be measured in animal by measuring the tumor size reduction over a period of time, for example, 6 month for human and 4-6 weeks for mouse. A composition or a method sensitizes response to a therapeutic treatment if the increase in treatment sensitivity or the reduction in resistance is 25% or more, for example, 30%, 40%, 50%, 60%, 70%, 80%, or more, to 2-fold, 3-fold, 4-fold, 5-fold, 10-fold, 15-fold, 20-fold or more, compared to treatment sensitivity or resistance in the absence of such composition or method. The determination of sensitivity or resistance to a therapeutic treatment is routine in the art and within the skill of an ordinarily skilled clinician. It is to be understood that any method described herein for enhancing the efficacy of a cancer therapy can be equally applied to methods for sensitizing hyperproliferative or otherwise cancerous cells (*e.g.,* resistant cells) to the cancer therapy.

The term "synergistic effect" refers to the combined effect of two or more anti-immune checkpoint agents can be greater than the sum of the separate effects of the anticancer agents alone.

"Short interfering RNA" (siRNA), also referred to herein as "small interfering *R*NA" is defined as an agent which functions to inhibit expression of a target biomarker nucleic acid, *e.g.,* by RNAi. An siRNA may be chemically synthesized, may be produced by *in vitro* transcription, or may be produced within a host cell. In one embodiment, siRNA is a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, or 22 nucleotides in length, and may contain a 3' and/or 5' overhang on each strand having a length of about 0, 1, 2, 3, 4, or 5 nucleotides. The length of the overhang is independent between the two strands, *i.e.,* the length of the overhang on one strand is not dependent on the length of the overhang on the second strand. Preferably the siRNA is capable of promoting RNA interference through degradation or specific post-transcriptional gene silencing (PTGS) of the target messenger RNA (mRNA).

In another embodiment, an siRNA is a small hairpin (also called stem loop) RNA (shRNA). In one embodiment, these shRNAs are composed of a short (*e.g.,* 19-25 nucleotide) antisense strand, followed by a 5-9 nucleotide loop, and the analogous sense strand. Alternatively, the sense strand may precede the nucleotide loop structure and the antisense strand may follow. These shRNAs may be contained in plasmids, retroviruses, and lentiviruses and expressed from, for example, the pol III U6 promoter, or another promoter (*see, e.g*.*,* Stewart, et al. (2003) RNA 9:493-501, incorporated by reference herein).

RNA interfering agents, *e.g.,* siRNA molecules, may be administered to a patient having or at risk for having cancer, to inhibit expression of a biomarker gene which is overexpressed in cancer and thereby treat, prevent, or inhibit cancer in the subject.

The term "subject" refers to any healthy animal, mammal or human, or any animal, mammal or human afflicted with a cancer, *e.g.,* lung, ovarian, pancreatic, liver, breast, prostate, and colon carcinomas, as well as melanoma and multiple myeloma. The term "subject" is interchangeable with "patient."

The term "survival" includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.* time of diagnosis or start of treatment) and end point (*e.g.* death, recurrence or metastasis). In addition, criteria for efficacy of treatment can be expanded to include response to chemotherapy, probability of survival, probability of metastasis within a given time period, and probability of tumor recurrence.

The term "therapeutic effect" refers to a local or systemic effect in animals, particularly mammals, and more particularly humans, caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a substance that produces some desired local or systemic effect at a reasonable benefit/risk ratio applicable to any treatment. In certain embodiments, a therapeutically effective amount of a compound will depend on its therapeutic index, solubility, and the like. For example, certain compounds discovered by the methods of the present invention may be administered in a sufficient amount to produce a reasonable benefit/risk ratio applicable to such treatment.

The terms "therapeutically-effective amount" and "effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment. Toxicity and therapeutic efficacy of subject compounds may be determined by standard pharmaceutical procedures in cell cultures or experimental animals, *e.g.,* for determining the LD₅₀ and the ED₅₀. Compositions that exhibit large therapeutic indices are preferred. In some embodiments, the LD₅₀ (lethal dosage) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more reduced for the agent relative to no administration of the agent. Similarly, the ED₅₀ (*i.e*., the concentration which achieves a half-maximal inhibition of symptoms) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more increased for the agent relative to no administration of the agent. Also, Similarly, the IC₅₀ (*i.e*., the concentration which achieves half-maximal cytotoxic or cytostatic effect on cancer cells) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more increased for the agent relative to no administration of the agent. In some embodiments, cancer cell growth in an assay can be inhibited by at least about 10%, 15%, 20%, 25%, 30%, 35%, 40%, 45%, 50%, 55%, 60%, 65%, 70%, 75%, 80%, 85%, 90%, 95%, or even 100%. In another embodiment, at least about a 10% , 15%, 20%, 25%, 30%, 35%, 40%, 45%, 50%, 55%, 60%, 65%, 70%, 75%, 80%, 85%, 90%, 95%, or even 100% decrease in a solid malignancy can be achieved.

A "transcribed polynucleotide" or "nucleotide transcript" is a polynucleotide (e.g. an mRNA, hnRNA, a cDNA, or an analog of such RNA or cDNA) which is complementary to or homologous with all or a portion of a mature mRNA made by transcription of a biomarker nucleic acid and normal post-transcriptional processing (*e.g.* splicing), if any, of the RNA transcript, and reverse transcription of the RNA transcript.

As used herein, the term "unresponsiveness" includes refractivity of immune cells to stimulation, *e.g.,* stimulation via an activating receptor or a cytokine. Unresponsiveness can occur, *e.g.,* because of exposure to immunosuppressants or exposure to high doses of antigen. As used herein, the term "anergy" or "tolerance" includes refractivity to activating receptor-mediated stimulation. Such refractivity is generally antigen-specific and persists after exposure to the tolerizing antigen has ceased. For example, anergy in T cells (as opposed to unresponsiveness) is characterized by lack of cytokine production, *e.g.,* IL-2. T cell anergy occurs when T cells are exposed to antigen and receive a first signal (a T cell receptor or CD-3 mediated signal) in the absence of a second signal (a costimulatory signal). Under these conditions, reexposure of the cells to the same antigen (even if reexposure occurs in the presence of a costimulatory polypeptide) results in failure to produce cytokines and, thus, failure to proliferate. Anergic T cells can, however, proliferate if cultured with cytokines (*e.g.,* IL-2). For example, T cell anergy can also be observed by the lack of IL-2 production by T lymphocytes as measured by ELISA or by a proliferation assay using an indicator cell line. Alternatively, a reporter gene construct can be used. For example, anergic T cells fail to initiate IL-2 gene transcription induced by a heterologous promoter under the control of the 5' IL-2 gene enhancer or by a multimer of the AP 1 sequence that can be found within the enhancer (Kang et al. (1992) Science 257:1134).

There is a known and definite correspondence between the amino acid sequence of a particular protein and the nucleotide sequences that can code for the protein, as defined by the genetic code (shown below). Likewise, there is a known and definite correspondence between the nucleotide sequence of a particular nucleic acid and the amino acid sequence encoded by that nucleic acid, as defined by the genetic code.

### GENETIC CODE

| | |
|---|---|
| Alanine (Ala, A) | GCA, GCC, GCG, GCT |
| Arginine (Arg, R) | AGA, ACG, CGA, CGC, CGG, CGT |
| Asparagine (Asn, N) | AAC, AAT |
| Aspartic acid (Asp, D) | GAC, GAT |
| Cysteine (Cys, C) | TGC, TGT |
| Glutamic acid (Glu, E) | GAA, GAG |
| Glutamine (Gln, Q) | CAA, CAG |
| Glycine (Gly, G) | GGA, GGC, GGG, GGT |
| Histidine (His, H) | CAC, CAT |
| Isoleucine (Ile, I) | ATA, ATC, ATT |
| Leucine (Leu, L) | CTA, CTC, CTG, CTT, TTA, TTG |
| Lysine (Lys, K) | AAA, AAG |
| Methionine (Met, M) | ATG |
| Phenylalanine (Phe, F) | TTC, TTT |
| Proline (Pro, P) | CCA, CCC, CCG, CCT |
| Serine (Ser, S) | AGC, AGT, TCA, TCC, TCG, TCT |
| Threonine (Thr, T) | ACA, ACC, ACG, ACT |
| Tryptophan (Trp, W) | TGG |
| Tyrosine (Tyr, Y) | TAC, TAT |
| Valine (Val, V) | GTA, GTC, GTG, GTT |
| Termination signal (end) | TAA, TAG, TGA |

An important and well known feature of the genetic code is its redundancy, whereby, for most of the amino acids used to make proteins, more than one coding nucleotide triplet may be employed (illustrated above). Therefore, a number of different nucleotide sequences may code for a given amino acid sequence. Such nucleotide sequences are considered functionally equivalent since they result in the production of the same amino acid sequence in all organisms (although certain organisms may translate some sequences more efficiently than they do others). Moreover, occasionally, a methylated variant of a purine or pyrimidine may be found in a given nucleotide sequence. Such methylations do not affect the coding relationship between the trinucleotide codon and the corresponding amino acid.

In view of the foregoing, the nucleotide sequence of a DNA or RNA encoding a biomarker nucleic acid (or any portion thereof) can be used to derive the polypeptide amino acid sequence, using the genetic code to translate the DNA or RNA into an amino acid sequence. Likewise, for polypeptide amino acid sequence, corresponding nucleotide sequences that can encode the polypeptide can be deduced from the genetic code (which, because of its redundancy, will produce multiple nucleic acid sequences for any given amino acid sequence). Thus, description and/or disclosure herein of a nucleotide sequence which encodes a polypeptide should be considered to also include description and/or disclosure of the amino acid sequence encoded by the nucleotide sequence. Similarly, description and/or disclosure of a polypeptide amino acid sequence herein should be considered to also include description and/or disclosure of all possible nucleotide sequences that can encode the amino acid sequence.

Finally, nucleic acid and amino acid sequence information for the loci and biomarkers useful for the present invention are well-known in the art and readily available on publicly available databases, such as the National Center for Biotechnology Information (NCBI).

### II. Subjects

In one embodiment, the subject for whom predicted likelihood of development of immune-related adverse events (irAE) is determined, is a mammal (*e.g.,* mouse, rat, primate, non-human mammal, domestic animal, such as a dog, cat, cow, horse, and the like), and is preferably a human.

In another embodiment of the methods of the present invention, the subject has not undergone treatment, such as chemotherapy, radiation therapy, targeted therapy, and/or immune checkpoint therapy. In still another embodiment, the subject has undergone treatment, such as chemotherapy, radiation therapy, targeted therapy, and/or immune checkpoint therapy.

In certain embodiments, the subject has had surgery to remove cancerous or precancerous tissue. In other embodiments, the cancerous tissue has not been removed, *e.g.,* the cancerous tissue may be located in an inoperable region of the body, such as in a tissue that is essential for life, or in a region where a surgical procedure would cause considerable risk of harm to the patient.

The methods of the present invention can be used to determine the development of immune-related adverse events (irAE) due to or associated with anti-immune checkpoint therapies of a cancer. In one embodiment, the cancer is one for which an immune checkpoint therapy (*e.g.,* anti-PD-1 blocking antibody, anti-PD-L1 blocking antibody, CTLA-4 blocking antibody, and the like) is FDA-approved for treatment, such as those described in the Examples. In one embodiment, the cancers are solid tumors, such as lung cancer such as non-small cell lung cancer, bladder cancer, melanoma such as metastatic melanoma, and/or renal cell carcinoma. In another embodiment, the cancer is an epithelial cancer such as, but not limited to, brain cancer (*e.g.,* glioblastomas) bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In still other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (*e.g.,* serous ovarian carcinoma), or breast carcinoma. The epithelial cancers may be characterized in various other ways including, but not limited to, serous, endometrioid, mucinous, clear cell, brenner, or undifferentiated. In yet other embodiments, the cancer is a mesenchymal cancer, such as sarcoma.

### III. Sample Collection, Preparation and Separation

In some embodiments, biomarker amount and/or activity measurement(s) in a sample from a subject is compared to a predetermined control (standard) sample. The sample from the subject is typically from a diseased tissue, such as cancer cells or tissues. The control sample can be from a different subject, from multiple subjects, or from a pre-compiled reference source. The control sample is typically a normal, non-diseased sample. However, in some embodiments, such as for staging of disease or for evaluating the efficacy of treatment, the control sample can be from a diseased tissue. The control sample can be a combination of samples from several different subjects. In some embodiments, the biomarker amount and/or activity measurement(s) from a subject is compared to a pre-determined level. This pre-determined level is typically obtained from normal samples. As described herein, a "pre-determined" biomarker amount and/or activity measurement(s) may be a biomarker amount and/or activity measurement(s) used to, by way of example only, evaluate a subject that may be selected for treatment, evaluate the likelihood of development of irAE due to an immune checkpoint therapy, and/or evaluate the likelihood of development of irAE due to a combination immune checkpoint therapy. A pre-determined biomarker amount and/or activity measurement(s) may be determined in populations of patients with or without cancer. The pre-determined biomarker amount and/or activity measurement(s) can be a single number, equally applicable to every patient, or the pre-determined biomarker amount and/or activity measurement(s) can vary according to specific subpopulations of patients. Age, weight, height, and other factors of a subject may affect the pre-determined biomarker amount and/or activity measurement(s) of the individual. Furthermore, the pre-determined biomarker amount and/or activity can be determined for each subject individually. In one embodiment, the amounts determined and/or compared in a method described herein are based on absolute measurements.

In another embodiment, the amounts determined and/or compared in a method described herein are based on relative measurements, such as ratios (*e.g.,* biomarker measurements relative to the expression of a housekeeping gene, and the like). Pretreatment biomarker measurement can be made at any time prior to initiation of anti-cancer therapy. Post-treatment biomarker measurement can be made at any time after initiation of anti-cancer therapy. In some embodiments, post-treatment biomarker measurements are made 1, 2, 3, 4, 5, 6, 7, 8, 9, 10, 11, 12, 13, 14, 15, 16, 17, 18, 19, 20 weeks or more after initiation of anti-cancer therapy, and even longer toward indefinitely for continued monitoring. Treatment can comprise anti-cancer therapy, such as a therapeutic regimen comprising an anti-PD1 monoclonal antibody (*e.g.,* nivolumab) alone or in combination with other anti-cancer agents, such as anti-PD-L1/PD-L2 antibodies, anti-VEGF agents (*e.g.,* bevacizumab), agents described in the Examples, Figures, and Tables.

The pre-determined biomarker amount and/or activity measurement(s) can be any suitable standard. For example, the pre-determined biomarker amount and/or activity measurement(s) can be obtained from the same or a different human for whom a patient selection is being assessed. In one embodiment, the pre-determined biomarker amount and/or activity measurement(s) can be obtained from a previous assessment of the same patient. In such a manner, the progress of the selection of the patient can be monitored over time. In addition, the control can be obtained from an assessment of another human or multiple humans, *e.g.,* selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group.

In some embodiments of the present invention the difference of biomarker amount and/or activity measurement(s) from the pre-determined level is about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 fold or greater, or any range in between, inclusive. Such cutoff values apply equally when the measurement is based on relative changes.

Biological samples can be collected from a variety of sources from a patient including a body fluid sample, cell sample, or a tissue sample comprising nucleic acids and/or proteins. "Body fluids" refer to fluids that are excreted or secreted from the body as well as fluids that are normally not (*e.g.,* amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit). In a preferred embodiment, the subject and/or control sample is selected from the group consisting of cells, cell lines, histological slides, paraffin embedded tissues, biopsies, whole blood, nipple aspirate, serum, plasma, buccal scrape, saliva, cerebrospinal fluid, urine, stool, and bone marrow. In one embodiment, the sample is serum, plasma, or urine. In another embodiment, the sample is serum.

The samples can be collected from individuals repeatedly over a longitudinal period of time (*e.g.,* once or more on the order of days, weeks, months, annually, biannually, etc.). Obtaining numerous samples from an individual over a period of time can be used to verify results from earlier detections and/or to identify an alteration in biological pattern as a result of, for example, disease progression, drug treatment, *etc.* For example, subject samples can be taken and monitored every month, every two months, or combinations of one, two, or three month intervals according to the present invention. In addition, the biomarker amount and/or activity measurements of the subject obtained over time can be conveniently compared with each other, as well as with those of normal controls during the monitoring period, thereby providing the subject's own values, as an internal, or personal, control for long-term monitoring.

Sample preparation and separation can involve any of the procedures, depending on the type of sample collected and/or analysis of biomarker measurement(s). Such procedures include, by way of example only, concentration, dilution, adjustment of pH, removal of high abundance polypeptides (*e.g.,* albumin, gamma globulin, and transferrin, etc.), addition of preservatives and calibrants, addition of protease inhibitors, addition of denaturants, desalting of samples, concentration of sample proteins, extraction and purification of lipids.

The sample preparation can also isolate molecules that are bound in non-covalent complexes to other protein (*e.g.,* carrier proteins). This process may isolate those molecules bound to a specific carrier protein (*e.g.,* albumin), or use a more general process, such as the release of bound molecules from all carrier proteins via protein denaturation, for example using an acid, followed by removal of the carrier proteins.

Removal of undesired proteins (*e.g.,* high abundance, uninformative, or undetectable proteins) from a sample can be achieved using high affinity reagents, high molecular weight filters, ultracentrifugation and/or electrodialysis. High affinity reagents include antibodies or other reagents (*e.g.,* aptamers) that selectively bind to high abundance proteins. Sample preparation could also include ion exchange chromatography, metal ion affinity chromatography, gel filtration, hydrophobic chromatography, chromatofocusing, adsorption chromatography, isoelectric focusing and related techniques. Molecular weight filters include membranes that separate molecules on the basis of size and molecular weight. Such filters may further employ reverse osmosis, nanofiltration, ultrafiltration and microfiltration.

Ultracentrifugation is a method for removing undesired polypeptides from a sample. Ultracentrifugation is the centrifugation of a sample at about 15,000-60,000 rpm while monitoring with an optical system the sedimentation (or lack thereof) of particles. Electrodialysis is a procedure which uses an electromembrane or semipermable membrane in a process in which ions are transported through semi-permeable membranes from one solution to another under the influence of a potential gradient. Since the membranes used in electrodialysis may have the ability to selectively transport ions having positive or negative charge, reject ions of the opposite charge, or to allow species to migrate through a semipermable membrane based on size and charge, it renders electrodialysis useful for concentration, removal, or separation of electrolytes.

Separation and purification in the present invention may include any procedure known in the art, such as capillary electrophoresis (*e.g.,* in capillary or on-chip) or chromatography (*e.g.,* in capillary, column or on a chip). Electrophoresis is a method which can be used to separate ionic molecules under the influence of an electric field. Electrophoresis can be conducted in a gel, capillary, or in a microchannel on a chip. Examples of gels used for electrophoresis include starch, acrylamide, polyethylene oxides, agarose, or combinations thereof. A gel can be modified by its cross-linking, addition of detergents, or denaturants, immobilization of enzymes or antibodies (affinity electrophoresis) or substrates (zymography) and incorporation of a pH gradient. Examples of capillaries used for electrophoresis include capillaries that interface with an electrospray.

Capillary electrophoresis (CE) is preferred for separating complex hydrophilic molecules and highly charged solutes. CE technology can also be implemented on microfluidic chips. Depending on the types of capillary and buffers used, CE can be further segmented into separation techniques such as capillary zone electrophoresis (CZE), capillary isoelectric focusing (CIEF), capillary isotachophoresis (cITP) and capillary electrochromatography (CEC). An embodiment to couple CE techniques to electrospray ionization involves the use of volatile solutions, for example, aqueous mixtures containing a volatile acid and/or base and an organic such as an alcohol or acetonitrile.

Capillary isotachophoresis (cITP) is a technique in which the analytes move through the capillary at a constant speed but are nevertheless separated by their respective mobilities. Capillary zone electrophoresis (CZE), also known as free-solution CE (FSCE), is based on differences in the electrophoretic mobility of the species, determined by the charge on the molecule, and the frictional resistance the molecule encounters during migration which is often directly proportional to the size of the molecule. Capillary isoelectric focusing (CIEF) allows weakly-ionizable amphoteric molecules, to be separated by electrophoresis in a pH gradient. CEC is a hybrid technique between traditional high performance liquid chromatography (HPLC) and CE.

Separation and purification techniques used in the present invention include any chromatography procedures known in the art. Chromatography can be based on the differential adsorption and elution of certain analytes or partitioning of analytes between mobile and stationary phases. Different examples of chromatography include, but not limited to, liquid chromatography (LC), gas chromatography (GC), high performance liquid chromatography (HPLC), etc.

### IV. Biomarkers

One aspect of the present invention pertains to the use of germline variations as biomarkers. As used herein, the term "nucleic acid molecule" is intended to include DNA molecules (*e.g.,* cDNA or genomic DNA) and RNA molecules (*e.g.,* mRNA) and analogs of the DNA or RNA generated using nucleotide analogs. The nucleic acid molecule can be single-stranded or double-stranded, but preferably is double-stranded DNA.

An "isolated" molecule is one which is separated from other molecules which are present in the natural source of the compound molecule. A biomarker molecule of the present invention can be isolated and/or purified using standard molecular biology techniques described herein.

A "copy number variation (CNV)" or a "copy number alteration (CNA)" results from a gene having a different number of copies between individuals. The CNV or CNA is not restricted to the coding sequence of the gene but may include any part of the gene of interest as well as a nearby promoter or enhancer that regulates the gene of interest.

As used herein, the term "copy number amplification" refers to a copy number above the standard copy number in the genome of a species, such as diploid in humans. In some embodiments, copy number amplification can be 1, 2, 3, 4, 5, 6, 7, 8, 9, 10, or more copies in addition to the standard copy number in the genome of the species.

As used herein, the term "copy number deletion" refers to a copy number below the standard copy number in the genome of a species, such as diploid in humans. In some embodiments, copy number amplification can be 1, 2, 3, 4, 5, 6, 7, 8, 9, 10, or more copies below the standard copy number in the genome of the species.

Similarly, a "single nucleotide variation (SNV)" refers to a variation at a single nucleotide position and includes, without limitation this includes "truncating" mutations that truncate or shorten the resulting protein, nonsynonymous mutations that alter the protein, or splice variant mutations that alter the splicing/exon composition of the resulting protein.

Germline mutations can be detected using various DNA sequencing techniques, such as next-generation sequencing. In particular, whole exon sequencing can be used to detect germline mutations. Additionally, microarrays may be used for detecting germline mutations. In some embodiments, germline mutations can be inferred from sequencing of tumor samples. In applications where only tumor DNA is available, germline mutations/variants that overlap known common variants in the population can be inferred probabilistically from a low number of sequencing reads and then refined (imputed) using an external reference panel of high-quality germline haplotypes. The presence of a germline variant "imputed" from tumors can then be used as an irAE biomarker as if it had been genotyped directly in the germline.

As used herein, "liquid chromatography" (LC) means a process of selective retardation of one or more components of a fluid solution as the fluid uniformly percolates through a column of a finely divided substance, or through capillary passageways. The retardation results from the distribution of the components of the mixture between one or more stationary phases and the bulk fluid, (*i.e.,* mobile phase), as this fluid moves relative to the stationary phase(s). "Liquid chromatography" includes reverse phase liquid chromatography (RPLC), high performance liquid chromatography (HPLC) and high turbulence liquid chromatography (HTLC).

As used herein, the term "HPLC" or "high performance liquid chromatography" refers to liquid chromatography in which the degree of separation is increased by forcing the mobile phase under pressure through a stationary phase, typically a densely packed column.

As used herein, the term "gas chromatography" refers to chromatography in which the sample mixture is vaporized and injected into a stream of carrier gas (as nitrogen or helium) moving through a column containing a stationary phase composed of a liquid or a particulate solid and is separated into its component compounds according to the affinity of the compounds for the stationary phase

As used herein, "mass spectrometry" (MS) refers to an analytical technique to identify compounds by their mass. MS technology generally includes (1) ionizing the compounds to form charged compounds; and (2) detecting the molecular weight of the charged compound and calculating a mass-to-charge ratio (m/z). The compound may be ionized and detected by any suitable means. A "mass spectrometer" generally includes an ionizer and an ion detector. See, *e.g.,* U.S. Pat. Nos. 6,204,500, entitled "Mass Spectrometry From Surfaces;" 6,107,623, entitled "Methods and Apparatus for Tandem Mass Spectrometry;" 6,268,144, entitled "DNA Diagnostics Based On Mass Spectrometry;" 6,124,137, entitled "Surface-Enhanced Photolabile Attachment And Release For Desorption And Detection Of Analytes;" Wright et al. (1999) Prostate Cancer Prostatic Dis 2:264-276; and Merchant and Weinberger (2000) Electrophoresis 21:1164-1167.

Biomarkers, such as those shown in Table 1, examples, and figures, can be detected in numerous ways according to well-known techniques. For example, one may use mass spectrometry methods, such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (*e.g.,* MS/MS, MS/MS/MS, ESI-MS/MS, etc.). See for example, U.S. Patent Application Nos: 20030199001, 20030134304, 20030077616, which are herein incorporated by reference.

Mass spectrometry methods are well known in the art and have been used to quantify and/or identify biomolecules, such as chemical metabolites and proteins (see, e.g., Li et al. (2000) Tibtech 18, 151-160; Rowley et al. (2000) Methods 20, 383-397; Kuster and Mann (1998) Curr. Opin. Structural Biol. 8, 393-400). Further, mass spectrometric techniques have been developed that permit at least partial *de novo* sequencing of isolated proteins (see, *e.g.,* Chait et al. (1993) Science 262, 89-92; Keough et al. (1999) Proc. Natl. Acad. Sci. USA. 96, 7131-7136; reviewed in Bergman (2000) EXS 88, 133-44).

In certain embodiments, a gas phase ion spectrophotometer is used. In other embodiments, laser-desorption/ionization mass spectrometry is used to analyze the sample. Modem laser desorption/ionization mass spectrometry ("LDI-MS") can be practiced in two main variations: matrix assisted laser desorption/ionization ("MALDI") mass spectrometry and surface-enhanced laser desorption/ionization ("SELDI"). In MALDI, the analyte is mixed with a solution containing a matrix, and a drop of the liquid is placed on the surface of a substrate. The matrix solution then co-crystallizes with the biological molecules. The substrate is inserted into the mass spectrometer. Laser energy is directed to the substrate surface where it desorbs and ionizes the biological molecules without significantly fragmenting them. However, MALDI has limitations as an analytical tool. It does not provide means for fractionating the sample, and the matrix material can interfere with detection, especially for low molecular weight analytes (see, *e.g.,* Hellenkamp et al., U.S. Pat. No. 5,118,937 and Beavis and Chait, U.S. Pat. No. 5,045,694).

In SELDI, the substrate surface is modified so that it is an active participant in the desorption process. In one variant, the surface is derivatized with adsorbent and/or capture reagents that selectively bind the protein of interest. In another variant, the surface is derivatized with energy absorbing molecules that are not desorbed when struck with the laser. In another variant, the surface is derivatized with molecules that bind the protein of interest and that contain a photolytic bond that is broken upon application of the laser. In each of these methods, the derivatizing agent generally is localized to a specific location on the substrate surface where the sample is applied (see, *e.g.,* Hutchens and Yip, U.S. Pat. No. 5,719,060 and Hutchens and Yip, WO 98/59361). The two methods can be combined by, for example, using a SELDI affinity surface to capture an analyte and adding matrix-containing liquid to the captured analyte to provide the energy absorbing material.

For additional information regarding mass spectrometers, see, e.g., Principles of Instrumental Analysis, 3rd edition., Skoog, Saunders College Publishing, Philadelphia, 1985; and Kirk-Othmer Encyclopedia of Chemical Technology, 4.sup.th ed. Vol. 15 (John Wiley & Sons, New York 1995), pp. 1071-1094.

Detection of the presence of a marker or other substances will typically involve detection of signal intensity. For example, in certain embodiments, the signal strength of peak values from spectra of a first sample and a second sample can be compared (e.g., visually or by computer analysis) to determine the relative amounts of particular biomolecules. Software programs such as the Biomarker Wizard program (Ciphergen Biosystems, Inc., Fremont, Calif.) can be used to aid in analyzing mass spectra. The mass spectrometers and their techniques are well known to those of skill in the art.

A person skilled in the art understands that any of the components of a mass spectrometer (*e.g.,* desorption source, mass analyzer, detect, etc.) and varied sample preparations can be combined with other suitable components or preparations described herein, or to those known in the art. For example, in some embodiments a control sample may contain heavy atoms (*e.g.* ¹³C) thereby permitting the test sample to be mixed with the known control sample in the same mass spectrometry run. In some embodiments, internal controls, such as phenylalanine-d8 and/or valine-d8 can be run with the samples.

In one embodiment, a laser desorption time-of-flight (TOF) mass spectrometer is used. In laser desorption mass spectrometry, a substrate with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio.

In some embodiments the relative amounts of one or more biomolecules present in a first or second sample is determined, in part, by executing an algorithm with a programmable digital computer. The algorithm identifies at least one peak value in the first mass spectrum and the second mass spectrum. The algorithm then compares the signal strength of the peak value of the first mass spectrum to the signal strength of the peak value of the second mass spectrum of the mass spectrum. The relative signal strengths are an indication of the amount of the biomolecule that is present in the first and second samples. A standard containing a known amount of a biomolecule can be analyzed as the second sample to provide better quantification of the amount of the biomolecule present in the first sample. In certain embodiments, the identity of the biomolecules in the first and second sample can also be determined.

### Allelic variations

In some embodiments, the biomarkers comprise one or more allelic variations, such as single nucleotide polymorphisms. Such biomarkers can be detected through various suitable techniques. For example, one may use DNA sequencing methods to identify allelic variations. In particular, one may use whole exome sequencing to identify allelic variations, such as single nucleotide polymorphisms.

### V. Anti-Cancer Therapies

The likelihood of development of immune-related adverse events (irAE), and thus efficacy of immune checkpoint therapy is predicted according to biomarker amount and/or activity associated with a cancer in a subject according to the methods described herein. In one embodiment, such immune checkpoint therapy or combinations of therapies (*e.g.,* anti-PD-1 antibodies) can be administered once a subject is indicated as being a likely responder to immune checkpoint therapy (*e.g.,* due to a low likelihood of developing irAE). In another embodiment, such immune checkpoint therapy can be avoided once a subject is indicated as not being a likely responder to immune checkpoint therapy and an alternative treatment regimen, such as targeted and/or untargeted anti-cancer therapies can be administered. Combination therapies are also contemplated and can comprise, for example, one or more chemotherapeutic agents and radiation, one or more chemotherapeutic agents and immunotherapy, or one or more chemotherapeutic agents, radiation and chemotherapy, each combination of which can be with immune checkpoint therapy.

The term "targeted therapy" refers to administration of agents that selectively interact with a chosen biomolecule to thereby treat cancer.

Immunotherapy is one form of targeted therapy that may comprise, for example, the use of cancer vaccines and/or sensitized antigen presenting cells. For example, an oncolytic virus is a virus that is able to infect and lyse cancer cells, while leaving normal cells unharmed, making them potentially useful in cancer therapy. Replication of oncolytic viruses both facilitates tumor cell destruction and also produces dose amplification at the tumor site. They may also act as vectors for anticancer genes, allowing them to be specifically delivered to the tumor site. The immunotherapy can involve passive immunity for short-term protection of a host, achieved by the administration of pre-formed antibody directed against a cancer antigen or disease antigen (*e.g.,* administration of a monoclonal antibody, optionally linked to a chemotherapeutic agent or toxin, to a tumor antigen). Immunotherapy can also focus on using the cytotoxic lymphocyte-recognized epitopes of cancer cell lines. Alternatively, antisense polynucleotides, ribozymes, RNA interference molecules, triple helix polynucleotides and the like, can be used to selectively modulate biomolecules that are linked to the initiation, progression, and/or pathology of a tumor or cancer.

The term "untargeted therapy" referes to administration of agents that do not selectively interact with a chosen biomolecule yet treat cancer. Representative examples of untargeted therapies include, without limitation, chemotherapy, gene therapy, and radiation therapy.

In one embodiment, chemotherapy is used. Chemotherapy includes the administration of a chemotherapeutic agent. Such a chemotherapeutic agent may be, but is not limited to, those selected from among the following groups of compounds: platinum compounds, cytotoxic antibiotics, antimetabolities, anti-mitotic agents, alkylating agents, arsenic compounds, DNA topoisomerase inhibitors, taxanes, nucleoside analogues, plant alkaloids, and toxins; and synthetic derivatives thereof. Exemplary compounds include, but are not limited to, alkylating agents: cisplatin, treosulfan, and trofosfamide; plant alkaloids: vinblastine, paclitaxel, docetaxol; DNA topoisomerase inhibitors: teniposide, crisnatol, and mitomycin; anti-folates: methotrexate, mycophenolic acid, and hydroxyurea; pyrimidine analogs: 5-fluorouracil, doxifluridine, and cytosine arabinoside; purine analogs: mercaptopurine and thioguanine; DNA antimetabolites: 2'-deoxy-5-fluorouridine, aphidicolin glycinate, and pyrazoloimidazole; and antimitotic agents: halichondrin, colchicine, and rhizoxin. Compositions comprising one or more chemotherapeutic agents (*e.g.,* FLAG, CHOP) may also be used. FLAG comprises fludarabine, cytosine arabinoside (Ara-C) and G-CSF. CHOP comprises cyclophosphamide, vincristine, doxorubicin, and prednisone. In another embodiments, PARP (*e.g.,* PARP-1 and/or PARP-2) inhibitors are used and such inhibitors are well known in the art (*e.g*., Olaparib, ABT-888, BSI-201, BGP-15 (N-Gene Research Laboratories, Inc.); INO-1001 (Inotek Pharmaceuticals Inc.); PJ34 (Soriano *et al.,* 2001; Pacher *et al.,* 2002b); 3-aminobenzamide (Trevigen); 4-amino-1,8-naphthalimide; (Trevigen); 6(5H)-phenanthridinone (Trevigen); benzamide (U.S. Pat. Re. 36,397); and NU1025 (Bowman et al.). The mechanism of action is generally related to the ability of PARP inhibitors to bind PARP and decrease its activity. PARP catalyzes the conversion of beta. -nicotinamide adenine dinucleotide (NAD+) into nicotinamide and poly-ADP-ribose (PAR). Both poly (ADP-ribose) and PARP have been linked to regulation of transcription, cell proliferation, genomic stability, and carcinogenesis (Bouchard V. J. et.al. Experimental Hematology, Volume 31, Number 6, June 2003, pp. 446-454(9); Herceg Z.; Wang Z.-Q. Mutation Research/Fundamental and Molecular Mechanisms of Mutagenesis, Volume 477, Number 1, 2 Jun. 2001, pp. 97-110(14)). Poly(ADP-ribose) polymerase 1 (PARP1) is a key molecule in the repair of DNA single-strand breaks (SSBs) (de Murcia J. et al. 1997. Proc Natl Acad Sci USA 94:7303-7307; Schreiber V, Dantzer F, Ame J C, de Murcia G (2006) Nat Rev Mol Cell Biol 7:517-528; Wang Z Q, et al. (1997) Genes Dev 11:2347-2358). Knockout of SSB repair by inhibition of PARP1 function induces DNA double-strand breaks (DSBs) that can trigger synthetic lethality in cancer cells with defective homology-directed DSB repair (Bryant H E, et al. (2005) Nature 434:913-917; Farmer H, et al. (2005) Nature 434:917-921). The foregoing examples of chemotherapeutic agents are illustrative, and are not intended to be limiting.

In another embodiment, radiation therapy is used. The radiation used in radiation therapy can be ionizing radiation. Radiation therapy can also be gamma rays, X-rays, or proton beams. Examples of radiation therapy include, but are not limited to, external-beam radiation therapy, interstitial implantation of radioisotopes (I-125, palladium, iridium), radioisotopes such as strontium-89, thoracic radiation therapy, intraperitoneal P-32 radiation therapy, and/or total abdominal and pelvic radiation therapy. For a general overview of radiation therapy, see Hellman, Chapter 16: Principles of Cancer Management: Radiation Therapy, 6th edition, 2001, DeVita et al., eds., J. B. Lippencott Company, Philadelphia. The radiation therapy can be administered as external beam radiation or teletherapy wherein the radiation is directed from a remote source. The radiation treatment can also be administered as internal therapy or brachytherapy wherein a radioactive source is placed inside the body close to cancer cells or a tumor mass. Also encompassed is the use of photodynamic therapy comprising the administration of photosensitizers, such as hematoporphyrin and its derivatives, Vertoporfin (BPD-MA), phthalocyanine, photosensitizer Pc4, demethoxy-hypocrellin A; and 2BA-2-DMHA.

In another embodiment, hormone therapy is used. Hormonal therapeutic treatments can comprise, for example, hormonal agonists, hormonal antagonists (*e.g.,* flutamide, bicalutamide, tamoxifen, raloxifene, leuprolide acetate (LUPRON), LH-RH antagonists), inhibitors of hormone biosynthesis and processing, and steroids (*e.g.,* dexamethasone, retinoids, deltoids, betamethasone, cortisol, cortisone, prednisone, dehydrotestosterone, glucocorticoids, mineralocorticoids, estrogen, testosterone, progestins), vitamin A derivatives (*e.g.,* all-trans retinoic acid (ATRA)); vitamin D3 analogs; antigestagens (*e.g.,* mifepristone, onapristone), or antiandrogens (*e.g.,* cyproterone acetate).

In another embodiment, hyperthermia, a procedure in which body tissue is exposed to high temperatures (up to 106°F.) is used. Heat may help shrink tumors by damaging cells or depriving them of substances they need to live. Hyperthermia therapy can be local, regional, and whole-body hyperthermia, using external and internal heating devices. Hyperthermia is almost always used with other forms of therapy (*e.g.,* radiation therapy, chemotherapy, and biological therapy) to try to increase their effectiveness. Local hyperthermia refers to heat that is applied to a very small area, such as a tumor. The area may be heated externally with high-frequency waves aimed at a tumor from a device outside the body. To achieve internal heating, one of several types of sterile probes may be used, including thin, heated wires or hollow tubes filled with warm water; implanted microwave antennae; and radiofrequency electrodes. In regional hyperthermia, an organ or a limb is heated. Magnets and devices that produce high energy are placed over the region to be heated. In another approach, called perfusion, some of the patient's blood is removed, heated, and then pumped (perfused) into the region that is to be heated internally. Whole-body heating is used to treat metastatic cancer that has spread throughout the body. It can be accomplished using warm-water blankets, hot wax, inductive coils (like those in electric blankets), or thermal chambers (similar to large incubators). Hyperthermia does not cause any marked increase in radiation side effects or complications. Heat applied directly to the skin, however, can cause discomfort or even significant local pain in about half the patients treated. It can also cause blisters, which generally heal rapidly.

In still another embodiment, photodynamic therapy (also called PDT, photoradiation therapy, phototherapy, or photochemotherapy) is used for the treatment of some types of cancer. It is based on the discovery that certain chemicals known as photosensitizing agents can kill one-celled organisms when the organisms are exposed to a particular type of light. PDT destroys cancer cells through the use of a fixed-frequency laser light in combination with a photosensitizing agent. In PDT, the photosensitizing agent is injected into the bloodstream and absorbed by cells all over the body. The agent remains in cancer cells for a longer time than it does in normal cells. When the treated cancer cells are exposed to laser light, the photosensitizing agent absorbs the light and produces an active form of oxygen that destroys the treated cancer cells. Light exposure must be timed carefully so that it occurs when most of the photosensitizing agent has left healthy cells but is still present in the cancer cells. The laser light used in PDT can be directed through a fiber-optic (a very thin glass strand). The fiber-optic is placed close to the cancer to deliver the proper amount of light. The fiber-optic can be directed through a bronchoscope into the lungs for the treatment of lung cancer or through an endoscope into the esophagus for the treatment of esophageal cancer. An advantage of PDT is that it causes minimal damage to healthy tissue. However, because the laser light currently in use cannot pass through more than about 3 centimeters of tissue (a little more than one and an eighth inch), PDT is mainly used to treat tumors on or just under the skin or on the lining of internal organs. Photodynamic therapy makes the skin and eyes sensitive to light for 6 weeks or more after treatment. Patients are advised to avoid direct sunlight and bright indoor light for at least 6 weeks. If patients must go outdoors, they need to wear protective clothing, including sunglasses. Other temporary side effects of PDT are related to the treatment of specific areas and can include coughing, trouble swallowing, abdominal pain, and painful breathing or shortness of breath. In December 1995, the U.S. Food and Drug Administration (FDA) approved a photosensitizing agent called porfimer sodium, or Photofrin^{®}, to relieve symptoms of esophageal cancer that is causing an obstruction and for esophageal cancer that cannot be satisfactorily treated with lasers alone. In January 1998, the FDA approved porfimer sodium for the treatment of early nonsmall cell lung cancer in patients for whom the usual treatments for lung cancer are not appropriate. The National Cancer Institute and other institutions are supporting clinical trials (research studies) to evaluate the use of photodynamic therapy for several types of cancer, including cancers of the bladder, brain, larynx, and oral cavity.

In yet another embodiment, laser therapy is used to harness high-intensity light to destroy cancer cells. This technique is often used to relieve symptoms of cancer such as bleeding or obstruction, especially when the cancer cannot be cured by other treatments. It may also be used to treat cancer by shrinking or destroying tumors. The term "laser" stands for light amplification by stimulated emission of radiation. Ordinary light, such as that from a light bulb, has many wavelengths and spreads in all directions. Laser light, on the other hand, has a specific wavelength and is focused in a narrow beam. This type of high-intensity light contains a lot of energy. Lasers are very powerful and may be used to cut through steel or to shape diamonds. Lasers also can be used for very precise surgical work, such as repairing a damaged retina in the eye or cutting through tissue (in place of a scalpel). Although there are several different kinds of lasers, only three kinds have gained wide use in medicine: Carbon dioxide (CO₂) laser--This type of laser can remove thin layers from the skin's surface without penetrating the deeper layers. This technique is particularly useful in treating tumors that have not spread deep into the skin and certain precancerous conditions. As an alternative to traditional scalpel surgery, the CO₂ laser is also able to cut the skin. The laser is used in this way to remove skin cancers. Neodymium:yttrium-aluminum-garnet (Nd:YAG) laser-- Light from this laser can penetrate deeper into tissue than light from the other types of lasers, and it can cause blood to clot quickly. It can be carried through optical fibers to less accessible parts of the body. This type of laser is sometimes used to treat throat cancers. Argon laser--This laser can pass through only superficial layers of tissue and is therefore useful in dermatology and in eye surgery. It also is used with light-sensitive dyes to treat tumors in a procedure known as photodynamic therapy (PDT). Lasers have several advantages over standard surgical tools, including: Lasers are more precise than scalpels. Tissue near an incision is protected, since there is little contact with surrounding skin or other tissue. The heat produced by lasers sterilizes the surgery site, thus reducing the risk of infection. Less operating time may be needed because the precision of the laser allows for a smaller incision. Healing time is often shortened; since laser heat seals blood vessels, there is less bleeding, swelling, or scarring. Laser surgery may be less complicated. For example, with fiber optics, laser light can be directed to parts of the body without making a large incision. More procedures may be done on an outpatient basis. Lasers can be used in two ways to treat cancer: by shrinking or destroying a tumor with heat, or by activating a chemical--known as a photosensitizing agent--that destroys cancer cells. In PDT, a photosensitizing agent is retained in cancer cells and can be stimulated by light to cause a reaction that kills cancer cells. CO₂ and Nd:YAG lasers are used to shrink or destroy tumors. They may be used with endoscopes, tubes that allow physicians to see into certain areas of the body, such as the bladder. The light from some lasers can be transmitted through a flexible endoscope fitted with fiber optics. This allows physicians to see and work in parts of the body that could not otherwise be reached except by surgery and therefore allows very precise aiming of the laser beam. Lasers also may be used with low-power microscopes, giving the doctor a clear view of the site being treated. Used with other instruments, laser systems can produce a cutting area as small as 200 microns in diameter--less than the width of a very fine thread. Lasers are used to treat many types of cancer. Laser surgery is a standard treatment for certain stages of glottis (vocal cord), cervical, skin, lung, vaginal, vulvar, and penile cancers. In addition to its use to destroy the cancer, laser surgery is also used to help relieve symptoms caused by cancer (palliative care). For example, lasers may be used to shrink or destroy a tumor that is blocking a patient's trachea (windpipe), making it easier to breathe. It is also sometimes used for palliation in colorectal and anal cancer. Laser-induced interstitial thermotherapy (LITT) is one of the most recent developments in laser therapy. LITT uses the same idea as a cancer treatment called hyperthermia; that heat may help shrink tumors by damaging cells or depriving them of substances they need to live. In this treatment, lasers are directed to interstitial areas (areas between organs) in the body. The laser light then raises the temperature of the tumor, which damages or destroys cancer cells.

The duration and/or dose of treatment with anti-immune checkpoint therapies may vary according to the particular anti-immune checkpoint agent or combination thereof. An appropriate treatment time for a particular cancer therapeutic agent will be appreciated by the skilled artisan. The present invention contemplates the continued assessment of optimal treatment schedules for each cancer therapeutic agent, where the phenotype of the cancer of the subject as determined by the methods of the present invention is a factor in determining optimal treatment doses and schedules.

Any means for the introduction of a polynucleotide into mammals, human or non-human, or cells thereof may be adapted to the practice of this invention for the delivery of the various constructs of the present invention into the intended recipient. In one embodiment of the present invention, the DNA constructs are delivered to cells by transfection, *i.e.,* by delivery of "naked" DNA or in a complex with a colloidal dispersion system. A colloidal system includes macromolecule complexes, nanocapsules, microspheres, beads, and lipid-based systems including oil-in-water emulsions, micelles, mixed micelles, and liposomes. The preferred colloidal system of this invention is a lipid-complexed or liposome-formulated DNA. In the former approach, prior to formulation of DNA, *e.g.,* with lipid, a plasmid containing a transgene bearing the desired DNA constructs may first be experimentally optimized for expression (*e.g*., inclusion of an intron in the 5' untranslated region and elimination of unnecessary sequences (Felgner, et al., Ann NY Acad Sci 126-139, 1995). Formulation of DNA, *e.g.* with various lipid or liposome materials, may then be effected using known methods and materials and delivered to the recipient mammal. See, *e.g.,* Canonico et al, Am J Respir Cell Mol Biol 10:24-29, 1994; Tsan et al, Am J Physiol 268; Alton et al., Nat Genet. 5: 135-142, 1993 and U.S. patent No. 5,679,647 by Carson et al.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs, which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand. Naked DNA or DNA associated with a delivery vehicle, *e.g.,* liposomes, can be administered to several sites in a subject (see below).

Nucleic acids can be delivered in any desired vector. These include viral or non-viral vectors, including adenovirus vectors, adeno-associated virus vectors, retrovirus vectors, lentivirus vectors, and plasmid vectors. Exemplary types of viruses include HSV (herpes simplex virus), AAV (adeno associated virus), HIV (human immunodeficiency virus), BIV (bovine immunodeficiency virus), and MLV (murine leukemia virus). Nucleic acids can be administered in any desired format that provides sufficiently efficient delivery levels, including in virus particles, in liposomes, in nanoparticles, and complexed to polymers.

The nucleic acids encoding a protein or nucleic acid of interest may be in a plasmid or viral vector, or other vector as is known in the art. Such vectors are well known and any can be selected for a particular application. In one embodiment of the present invention, the gene delivery vehicle comprises a promoter and a demethylase coding sequence. Preferred promoters are tissue-specific promoters and promoters which are activated by cellular proliferation, such as the thymidine kinase and thymidylate synthase promoters. Other preferred promoters include promoters which are activatable by infection with a virus, such as the α- and β-interferon promoters, and promoters which are activatable by a hormone, such as estrogen. Other promoters which can be used include the Moloney virus LTR, the CMV promoter, and the mouse albumin promoter. A promoter may be constitutive or inducible.

In another embodiment, naked polynucleotide molecules are used as gene delivery vehicles, as described in WO 90/11092 and U.S. Patent 5,580,859. Such gene delivery vehicles can be either growth factor DNA or RNA and, in certain embodiments, are linked to killed adenovirus. Curiel et al., Hum. Gene. Ther. 3:147-154, 1992. Other vehicles which can optionally be used include DNA-ligand (Wu et al., J. Biol. Chem. 264:16985-16987, 1989), lipid-DNA combinations (Felgner et al., Proc. Natl. Acad. Sci. USA 84:7413 7417, 1989), liposomes (Wang et al., Proc. Natl. Acad. Sci. 84:7851-7855, 1987) and microprojectiles (Williams et al., Proc. Natl. Acad. Sci. 88:2726-2730, 1991).

A gene delivery vehicle can optionally comprise viral sequences such as a viral origin of replication or packaging signal. These viral sequences can be selected from viruses such as astrovirus, coronavirus, orthomyxovirus, papovavirus, paramyxovirus, parvovirus, picornavirus, poxvirus, retrovirus, togavirus or adenovirus. In a preferred embodiment, the growth factor gene delivery vehicle is a recombinant retroviral vector. Recombinant retroviruses and various uses thereof have been described in numerous references including, for example, Mann et al., Cell 33:153, 1983, Cane and Mulligan, Proc. Nat'l. Acad. Sci. USA 81:6349, 1984, Miller et al., Human Gene Therapy 1:5-14, 1990, U.S. Patent Nos. 4,405,712, 4,861,719, and 4,980,289, and PCT Application Nos. WO 89/02,468, WO 89/05,349, and WO 90/02,806. Numerous retroviral gene delivery vehicles can be utilized in the present invention, including for example those described in EP 0,415,731; WO 90/07936; WO 94/03622; WO 93/25698; WO 93/25234; U.S. Patent No. 5,219,740; WO 9311230; WO 9310218; Vile and Hart, Cancer Res. 53:3860-3864, 1993; Vile and Hart, Cancer Res. 53:962-967, 1993; Ram et al., Cancer Res. 53:83-88, 1993; Takamiya et al., J. Neurosci. Res. 33:493-503, 1992; Baba et al., J. Neurosurg. 79:729-735, 1993 (U.S. Patent No. 4,777,127, GB 2,200,651, EP 0,345,242 and WO91/02805).

Other viral vector systems that can be used to deliver a polynucleotide of the present invention have been derived from herpes virus, *e.g.,* Herpes Simplex Virus (U.S. Patent No. 5,631,236 by Woo et al., issued May 20, 1997 and WO 00/08191 by Neurovex), vaccinia virus (Ridgeway (1988) Ridgeway, "Mammalian expression vectors," In: Rodriguez R L, Denhardt D T, ed. Vectors: A survey of molecular cloning vectors and their uses. Stoneham: Butterworth,; Baichwal and Sugden (1986) "Vectors for gene transfer derived from animal DNA viruses: Transient and stable expression of transferred genes," In: Kucherlapati R, ed. Gene transfer. New York: Plenum Press; Coupar et al. (1988) Gene, 68:1-10), and several RNA viruses. Preferred viruses include an alphavirus, a poxivirus, an arena virus, a vaccinia virus, a polio virus, and the like. They offer several attractive features for various mammalian cells (Friedmann (1989) Science, 244:1275-1281; Ridgeway, 1988, supra; Baichwal and Sugden, 1986, supra; Coupar et al., 1988; Horwich et al.(1990) J.Virol., 64:642-650).

In other embodiments, target DNA in the genome can be manipulated using well-known methods in the art. For example, the target DNA in the genome can be manipulated by deletion, insertion, and/or mutation are retroviral insertion, artificial chromosome techniques, gene insertion, random insertion with tissue specific promoters, gene targeting, transposable elements and/or any other method for introducing foreign DNA or producing modified DNA/modified nuclear DNA. Other modification techniques include deleting DNA sequences from a genome and/or altering nuclear DNA sequences. Nuclear DNA sequences, for example, may be altered by site-directed mutagenesis.

In other embodiments, recombinant biomarker polypeptides, and fragments thereof, can be administered to subjects. In some embodiments, fusion proteins can be constructed and administered which have enhanced biological properties. In addition, the biomarker polypeptides, and fragment thereof, can be modified according to well-known pharmacological methods in the art (*e.g.,* pegylation, glycosylation, oligomerization, *etc.*) in order to further enhance desirable biological activities, such as increased bioavailability and decreased proteolytic degradation.

### VI. Clinical Efficacy

Clinical efficacy can be measured by any method known in the art. In the case of individual germline variants biomarkers for irAEs (*e.g.,* those in Table 1), a "genome-wide significance" cutoff was used in the working examples (and can be used in various embodiments). In some embodiments, a genome-wide significance cutoff may be a p-value of less than or equal to 5×10⁻², 10⁻², 5×10⁻³, 10⁻³, 5×10⁻⁴, 10⁻⁴, 5×10⁻⁵, 10⁻⁵, 5×10⁻⁶, 10⁻⁶, 5×10⁻⁷, 10⁻⁷, 5×10⁻⁸, 10⁻⁸, or less, or any range in between inclusive, such as 5×10⁻⁷ and 5×10⁻⁸. In some embodiments, a genome-wide significant cutoff is a p-value < 5×10⁻⁸ as used in the working examples described herein. A "nominal significance" cutoff can also be used, as demonstrated in the working examples (and can be used in various embodiments) to establish statistical significance and clinical efficacy. In some embodiments, a nominal significance cutoff may be a p-value of less than or equal to 5×10⁻², 10⁻², 5×10⁻³, 10⁻³, 5×10⁻⁴, 10⁻⁴, 5×10⁻⁵, 10⁻⁵, 5×10⁻⁶, 10⁻⁶, 5×10⁻⁷, 10⁻⁷, 5×10⁻⁸, 10⁻⁸, or less, or any range in between inclusive, such as 5×10⁻⁵ and 5×10⁻⁶. In some embodiments, a nominal significance cutoff is a p-value < 5×10⁻⁶ as used in the working examples described herein. In the case of polygenic risk score biomarkers, a minimum correlation coefficient (R²) can be used, as demonstrated in the working examples, to establish clinical efficacy. In the working examples, the statistical significance of the correlation coefficient was evaluated by linear/logistic regression or Cox regression and any features with nominally significant (as defined above) p-values were retained as biomarkers. In the case of somatic or germline variant biomarkers for irAEs, the hazard ratio (HR) of carriers versus non-carriers for time-to-irAE was used in the working examples (and can be used in various embodiments) to measure clinical efficacy. In the working examples, the significance of the hazard ratio (HR) was evaluated in a Cox regression model and any features with nominally significant (as defined above) hazard ratios were retained as biomarkers.

The response to a therapy, such as anti-immune checkpoint therapies, relates to any response of the cancer, *e.g.,* a tumor, to the therapy, preferably to a change in tumor mass and/or volume after initiation of neoadjuvant or adjuvant chemotherapy. Tumor response may be assessed in a neoadjuvant or adjuvant situation where the size of a tumor after systemic intervention can be compared to the initial size and dimensions as measured by CT, PET, mammogram, ultrasound or palpation and the cellularity of a tumor can be estimated histologically and compared to the cellularity of a tumor biopsy taken before initiation of treatment. Response may also be assessed by caliper measurement or pathological examination of the tumor after biopsy or surgical resection. Response may be recorded in a quantitative fashion like percentage change in tumor volume or cellularity or using a semi-quantitative scoring system such as residual cancer burden (Symmans et al., J. Clin. Oncol. (2007) 25:4414-4422) or Miller-Payne score (Ogston et al., (2003) Breast (Edinburgh, Scotland) 12:320-327) in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of tumor response may be performed early after the onset of neoadjuvant or adjuvant therapy, *e.g.,* after a few hours, days, weeks or preferably after a few months. A typical endpoint for response assessment is upon termination of neoadjuvant chemotherapy or upon surgical removal of residual tumor cells and/or the tumor bed. Similar approaches can be used for measuring immune-related adverse evets (irAE), which ultimately affect the efficacy of the treatment (e.g., immune-checkpoint therapy).

In some embodiments, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments, the CBR for a particular anti-immune checkpoint therapeutic regimen is at least 25%, 30%, 35%, 40%, 45%, 50%, 55%, 60%, 65%, 70%, 75%, 80%, 85%, or more.

Additional criteria for evaluating the likelihood of development of irAE due to anti-immune checkpoint therapies are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.,* time of diagnosis or start of treatment) and end point (*e.g.,* death, recurrence or metastasis). In addition, criteria for efficacy of treatment can be expanded to include response to chemotherapy, probability of survival, probability of metastasis within a given time period, and probability of tumor recurrence.

For example, in order to determine appropriate threshold values, a particular anti-immune checkpoint therapeutic regimen can be administered to a population of subjects and the outcome can be correlated to biomarker measurements that were determined prior to administration of any immune checkpoint therapy. The outcome measurement may be pathologic response to therapy given in the neoadjuvant setting. Alternatively, outcome measures, such as overall survival and disease-free survival can be monitored over a period of time for subjects following immune checkpoint therapy for whom biomarker measurement values are known. In certain embodiments, the same doses of anti-immune checkpoint agents are administered to each subject. In related embodiments, the doses administered are standard doses known in the art for anti-immune checkpoint agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker measurement threshold values that correlate to outcome of an immune checkpoint therapy can be determined using methods such as those described in the Examples section.

### VII. Further Uses and Methods of the Present Invention

The methods described herein can be used in a variety of diagnostic, prognostic, and therapeutic applications. In any method described herein, such as a diagnostic method, prognostic method, therapeutic method, or combination thereof, all steps of the method can be performed by a single actor or, alternatively, by more than one actor. For example, diagnosis can be performed directly by the actor providing therapeutic treatment. Alternatively, a person providing a therapeutic agent can request that a diagnostic assay be performed. The diagnostician and/or the therapeutic interventionist can interpret the diagnostic assay results to determine a therapeutic strategy. Similarly, such alternative processes can apply to other assays, such as prognostic assays. The compositions described herein can also be used in a variety of diagnostic, prognostic, and therapeutic applications regarding biomarkers described herein, such as those listed in Table 1. Moreover, any method of diagnosis, prognosis, prevention, and the like described herein can be be applied to a therapy or test agent of interest, such as immune checkpoint therapies, anti-cancer therapies, and the like.

### a. Screening Methods

One aspect of the present invention relates to screening assays, including non-cell based assays. In one embodiment, the assays provide a method for identifying whether a cancer is likely to respond to immune checkpoint therapy and/or whether an agent can inhibit the growth of or kill a cancer cell that is unlikely to respond to immune checkpoint therapy.

In one embodiment, the present invention relates to assays for screening test agents which modulates the amount of at least one biomarker listed in Table 1. In one embodiment, a method for identifying such an agent entails determining the ability of the agent to modulate, *e.g.* reduce, the amount of the at least one biomarker listed in Table 1. When the biomarker is a germline variant, given that it would be fixed at the DNA level, what is meant by modulation in the context of these screening methods is a change in the expression level or a change in an epigenetic attribute of the biomarker.

In one embodiment, an assay is a cell-free or cell-based assay, comprising contacting at least one biomarker listed in Table 1, with a test agent, and determining the ability of the test agent to modulate (*e.g.* reduce) the amount of the biomarker, such as by measuring direct binding of substrates or by measuring indirect parameters as described below.

For example, in a direct binding assay, biomarker molecules can be coupled with a radioisotope or enzymatic label such that binding can be determined by detecting the labeled protein or molecule in a complex. For example, the targets can be labeled with ¹²⁵I, ³⁵S, ¹⁴C, or ³H, either directly or indirectly, and the radioisotope detected by direct counting of radioemmission or by scintillation counting. Alternatively, the targets can be enzymatically labeled with, for example, horseradish peroxidase, alkaline phosphatase, or luciferase, and the enzymatic label detected by determination of conversion of an appropriate substrate to product. Determining the interaction between biomarker and substrate can also be accomplished using standard binding or enzymatic analysis assays. In one or more embodiments of the above described assay methods, it may be desirable to immobilize polypeptides or molecules to facilitate separation of complexed from uncomplexed forms of one or both of the proteins or molecules, as well as to accommodate automation of the assay.

Binding of a test agent to a target can be accomplished in any vessel suitable for containing the reactants. Non-limiting examples of such vessels include microtiter plates, test tubes, and micro-centrifuge tubes. Immobilized forms of the antibodies of the present invention can also include antibodies bound to a solid phase like a porous, microporous (with an average pore diameter less than about one micron) or macroporous (with an average pore diameter of more than about 10 microns) material, such as a membrane, cellulose, nitrocellulose, or glass fibers; a bead, such as that made of agarose or polyacrylamide or latex; or a surface of a dish, plate, or well, such as one made of polystyrene.

In an alternative embodiment, determining the ability of the agent to modulate the amount of the biomarker can be accomplished by determining the ability of the test agent to modulate the activity of a polypeptide or other product that functions downstream or upstream of its position within the signaling pathway (*e.g.,* feedback loops). Such feedback loops are well-known in the art (see, for example, Chen and Guillemin (2009) Int. J. Tryptophan Res. 2:1-19).

The present invention further pertains to novel agents identified by the above-described screening assays. Accordingly, it is within the scope of this invention to further use an agent identified as described herein in an appropriate animal model. For example, an agent identified as described herein can be used in an animal model to determine the efficacy, toxicity, or side effects of treatment with such an agent. Alternatively, an antibody identified as described herein can be used in an animal model to determine the mechanism of action of such an agent.

### 6. Predictive Medicine

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining the amount and/or activity level of a biomarker listed in Table 1 in the context of a biological sample (*e.g.,* blood, serum, cells, or tissue) to thereby determine whether an individual afflicted with a cancer is likely to respond to immune checkpoint therapy, whether in an original or recurrent cancer. Such assays can be used for prognostic or predictive purpose to thereby prophylactically treat an individual prior to the onset or after recurrence of a disorder characterized by or associated with biomarker polypeptide, nucleic acid expression or activity. The skilled artisan will appreciate that any method can use one or more (*e.g.,* combinations) of biomarkers listed in Table 1.

When the biomarker is a germline variant, given that it would be fixed at the DNA level, what is meant by modulation in the context of these predictive medicine methods is a change in the expression level or a change in an epigenetic attribute of the biomarker.

Another aspect of the present invention pertains to monitoring the influence of agents (*e.g.,* drugs, compounds, and small nucleic acid-based molecules) on the expression or activity of a biomarker listed in Table 1. These and other agents are described in further detail in the following sections.

The skilled artisan will also appreciate that, in certain embodiments, the methods of the present invention implement a computer program and computer system. For example, a computer program can be used to perform the algorithms described herein. A computer system can also store and manipulate data generated by the methods of the present invention which comprises a plurality of biomarker signal changes/profiles which can be used by a computer system in implementing the methods of this invention. In certain embodiments, a computer system receives biomarker expression data; (ii) stores the data; and (iii) compares the data in any number of ways described herein (*e.g.,* analysis relative to appropriate controls) to determine the state of informative biomarkers from cancerous or pre-cancerous tissue. In other embodiments, a computer system (i) compares the determined expression biomarker level to a threshold value; and (ii) outputs an indication of whether said biomarker level is significantly modulated (*e.g.,* above or below) the threshold value, or a phenotype based on said indication.

In certain embodiments, such computer systems are also considered part of the present invention. Numerous types of computer systems can be used to implement the analytic methods of this invention according to knowledge possessed by a skilled artisan in the bioinformatics and/or computer arts. Several software components can be loaded into memory during operation of such a computer system. The software components can comprise both software components that are standard in the art and components that are special to the present invention (*e.g.,* dCHIP software described in Lin et al. (2004) Bioinformatics 20, 1233-1240; radial basis machine learning algorithms (RBM) known in the art).

The methods of the present invention can also be programmed or modeled in mathematical software packages that allow symbolic entry of equations and high-level specification of processing, including specific algorithms to be used, thereby freeing a user of the need to procedurally program individual equations and algorithms. Such packages include, *e.g.,* Matlab from Mathworks (Natick, Mass.), Mathematica from Wolfram Research (Champaign, Ill.) or S-Plus from MathSoft (Seattle, Wash.).

In certain embodiments, the computer comprises a database for storage of biomarker data. Such stored profiles can be accessed and used to perform comparisons of interest at a later point in time. For example, biomarker production profiles of a sample derived from the non-cancerous tissue of a subject and/or profiles generated from population-based distributions of informative loci of interest in relevant populations of the same species can be stored and later compared to that of a sample derived from the cancerous tissue of the subject or tissue suspected of being cancerous of the subject.

In addition to the exemplary program structures and computer systems described herein, other, alternative program structures and computer systems will be readily apparent to the skilled artisan. Such alternative systems, which do not depart from the above described computer system and programs structures either in spirit or in scope, are therefore intended to be comprehended within the accompanying claims.

### c. Diagnostic Assays

The present invention provides, in part, methods, systems, and code for accurately classifying whether a biological sample is associated with a cancer that is likely to respond to immune checkpoint therapy. In some embodiments, the present invention is useful for classifying a sample (*e.g.,* from a subject) as associated with or at risk for responding to or not responding to immune checkpoint therapy using a statistical algorithm and/or empirical data (*e.g.,* the amount or activity of a biomarker listed in Table 1).

An exemplary method for detecting the amount or activity of a biomarker listed in Table 1, and thus useful for classifying whether a sample is likely or unlikely to respond to immune checkpoint therapy involves obtaining a biological sample from a test subject and contacting the biological sample with an agent, such as a protein-binding agent like an antibody or antigen-binding fragment thereof, or a nucleic acid-binding agent like an oligonucleotide, capable of detecting the amount or activity of the biomarker in the biological sample. In some embodiments, at least one antibody or antigen-binding fragment thereof is used, wherein two, three, four, five, six, seven, eight, nine, ten, or more such antibodies or antibody fragments can be used in combination (*e.g.,* in sandwich ELISAs) or in serial. In certain instances, the statistical algorithm is a single learning statistical classifier system. For example, a single learning statistical classifier system can be used to classify a sample as a based upon a prediction or probability value and the presence or level of the biomarker. The use of a single learning statistical classifier system typically classifies the sample as, for example, a likely immune checkpoint therapy responder or progressor sample with a sensitivity, specificity, positive predictive value, negative predictive value, and/or overall accuracy of at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Other suitable statistical algorithms are well known to those of skill in the art. For example, learning statistical classifier systems include a machine learning algorithmic technique capable of adapting to complex data sets (*e.g.,* panel of markers of interest) and making decisions based upon such data sets. In some embodiments, a single learning statistical classifier system such as a classification tree (*e.g.,* random forest) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.,* decision/classification trees such as random forests, classification and regression trees (C&RT), boosted trees, etc.), Probably Approximately Correct (PAC) learning, connectionist learning (*e.g.,* neural networks (NN), artificial neural networks (ANN), neuro fuzzy networks (NFN), network structures, perceptrons such as multi-layer perceptrons, multi-layer feed-forward networks, applications of neural networks, Bayesian learning in belief networks, etc.), reinforcement learning (*e.g.,* passive learning in a known environment such as naive learning, adaptive dynamic learning, and temporal difference learning, passive learning in an unknown environment, active learning in an unknown environment, learning action-value functions, applications of reinforcement learning, etc.), and genetic algorithms and evolutionary programming. Other learning statistical classifier systems include support vector machines (*e.g.,* Kernel methods), multivariate adaptive regression splines (MARS), Levenberg-Marquardt algorithms, Gauss-Newton algorithms, mixtures of Gaussians, gradient descent algorithms, and learning vector quantization (LVQ). In certain embodiments, the method of the present invention further comprises sending the sample classification results to a clinician, *e.g.,* an oncologist.

In another embodiment, the diagnosis of a subject is followed by administering to the individual a therapeutically effective amount of a defined treatment based upon the diagnosis.

In one embodiment, the methods further involve obtaining a control biological sample (*e.g.,* biological sample from a subject who does not have a cancer or whose cancer is susceptible to immune checkpoint therapy), a biological sample from the subject during remission, or a biological sample from the subject during treatment for developing a cancer progressing despite immune checkpoint therapy.

### d. Prognostic Assays

The diagnostic methods described herein can furthermore be utilized to identify subjects having or at risk of developing a cancer that is likely or unlikely to have immune-related adverse events associated with immune checkpoint therapy. The assays described herein, such as the preceding diagnostic assays or the following assays, can be utilized to identify a subject having or at risk of developing a disorder associated with a misregulation of the amount or activity of at least one biomarker described in Table 1, such as in cancer. When the biomarker is a germline variant, given that it would be fixed at the DNA level, what is meant by modulation in the context of these prognostic methods is a change in the expression level or a change in an epigenetic attribute of the biomarker. Alternatively, the prognostic assays can be utilized to identify a subject having or at risk for developing a disorder associated with a misregulation of the at least one biomarker described in Table 1, such as in cancer. Furthermore, the prognostic assays described herein can be used to determine whether a subject can be administered an agent (*e.g.,* an agonist, antagonist, peptidomimetic, polypeptide, peptide, nucleic acid, small molecule, or other drug candidate) to treat a disease or disorder associated with the aberrant biomarker expression or activity.

### e. Treatment Methods

The compositions described herein (including dual binding antibodies and derivatives and conjugates thereof) can be used in a variety of *in vitro* and *in vivo* therapeutic applications using the formulations and/or combinations described herein. In one embodiment, anti-immune checkpoint agents can be used to treat cancers of interest, such as those determined not to have immune-related adverse events (irAE) associated with immune checkpoint therapy. For example, antibodies that block the interaction between PD-L1, PD-L2, and/or CTLA-4 and their receptors (*e.g.,* PD-L1 binding to PD-1, PD-L2 binding to PD-1, and the like) can be used to treat cancer in subjects identified as not likely to develop irAE associated with immune checkpoint therapy. By contrast, alternative non-immune checkpoint therapies, such as standard of care chemotherapies, radiotherapies, and the like can be used to treat cancer in subjects identified as not likely to develop irAE associated with immune checkpoint therapy.

### 6. Pharmaceutical Compositions

In another aspect, the present invention provides pharmaceutically acceptable compositions which comprise a therapeutically-effective amount of an agent formulated together with one or more pharmaceutically acceptable carriers (additives) and/or diluents. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "therapeutically-effective amount" as used herein means that amount of an agent that modulates (*e.g.,* inhibits) biomarker expression and/or activity, or expression and/or activity of the complex, or composition comprising an agent that modulates (*e.g.,* inhibits) biomarker expression and/or activity, or expression and/or activity of the complex, which is effective for producing some desired therapeutic effect, *e.g.,* cancer treatment, at a reasonable benefit/risk ratio.

The phrase "pharmaceutically acceptable" is employed herein to refer to those agents, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically-acceptable salts" refers to the relatively non-toxic, inorganic and organic acid addition salts of the agents that modulates (e.g., inhibits) biomarker expression and/or activity, or expression and/or activity of the complex encompassed by the present invention. These salts can be prepared in situ during the final isolation and purification of the agents, or by separately reacting a purified agent in its free base form with a suitable organic or inorganic acid, and isolating the salt thus formed. ReRepresentative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, for example, Berge et al. (1977) "Pharmaceutical Salts", J. Pharm. Sci. 66: 1-19).

In other cases, the agents useful in the methods of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of agents that modulates (*e.g.,* inhibits) biomarker expression and/or activity, or expression and/or activity of the complex. These salts can likewise be prepared in situ during the final isolation and purification of the agents, or by separately reacting the purified agent in its free acid form with a suitable base, such as the hydroxide, carbonate or bicarbonate of a pharmaceutically-acceptable metal cation, with ammonia, or with a pharmaceutically-acceptable organic primary, secondary or tertiary amine. ReRepresentative alkali or alkaline earth salts include the lithium, sodium, potassium, calcium, magnesium, and aluminum salts and the like. ReRepresentative organic amines useful for the formation of base addition salts include ethylamine, diethylamine, ethylenediamine, ethanolamine, diethanolamine, piperazine and the like (see, for example, Berge *et al., supra*)*.*

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations useful in the methods of the present invention include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient, which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association an agent that modulates (*e.g.,* inhibits) biomarker expression and/or activity, with the carrier and, optionally, one or more accessory ingredients. In general, the formulations are prepared by uniformly and intimately bringing into association a agent with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a agent as an active ingredient. A compound may also be administered as a bolus, electuary or paste.

In solid dosage forms for oral administration (capsules, tablets, pills, dragees, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions, which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active agent may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing one or more agents with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Formulations which are suitable for vaginal administration also include pessaries, tampons, creams, gels, pastes, foams or spray formulations containing such carriers as are known in the art to be appropriate.

Dosage forms for the topical or transdermal administration of an agent that modulates (e.g., inhibits) biomarker expression and/or activity include powders, sprays, ointments, pastes, creams, lotions, gels, solutions, patches and inhalants. The active component may be mixed under sterile conditions with a pharmaceutically-acceptable carrier, and with any preservatives, buffers, or propellants which may be required.

The ointments, pastes, creams and gels may contain, in addition to a agent, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an agent that modulates (*e.g.,* inhibits) biomarker expression and/or activity, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The agent that modulates (*e.g.,* inhibits) biomarker expression and/or activity, can be alternatively administered by aerosol. This is accomplished by preparing an aqueous aerosol, liposomal preparation or solid particles containing the compound. A nonaqueous (*e.g.,* fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of a agent to the body. Such dosage forms can be made by dissolving or dispersing the agent in the proper medium. Absorption enhancers can also be used to increase the flux of the peptidomimetic across the skin. The rate of such flux can be controlled by either providing a rate controlling membrane or dispersing the peptidomimetic in a polymer matrix or gel.

Ophthalmic formulations, eye ointments, powders, solutions and the like, are also contemplated as being within the scope of this invention.

Pharmaceutical compositions of this invention suitable for parenteral administration comprise one or more agents in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the present invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of an agent that modulates (*e.g.,* inhibits) biomarker expression and/or activity, in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions, which are compatible with body tissue.

When the agents of the present invention are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

Actual dosage levels of the active ingredients in the pharmaceutical compositions of this invention may be determined by the methods of the present invention so as to obtain an amount of the active ingredient, which is effective to achieve the desired therapeutic response for a particular subject, composition, and mode of administration, without being toxic to the subject.

The nucleic acid molecules of the present invention can be inserted into vectors and used as gene therapy vectors. Gene therapy vectors can be delivered to a subject by, for example, intravenous injection, local administration (see U.S. Pat. No. 5,328,470) or by stereotactic injection (see *e.g.,* Chen et al. (1994) Proc. Natl. Acad. Sci. USA 91:3054 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

The present invention also encompasses kits for detecting and/or modulating biomarkers described herein. A kit of the present invention may also include instructional materials disclosing or describing the use of the kit or an antibody of the disclosed invention in a method of the disclosed invention as provided herein. A kit may also include additional components to facilitate the particular application for which the kit is designed. For example, a kit may additionally contain means of detecting the label (*e.g.,* enzyme substrates for enzymatic labels, filter sets to detect fluorescent labels, appropriate secondary labels such as a sheep anti-mouse-HRP, *etc.*) and reagents necessary for controls (*e.g.,* control biological samples or standards). A kit may additionally include buffers and other reagents recognized for use in a method of the disclosed invention. Non-limiting examples include agents to reduce non-specific binding, such as a carrier protein or a detergent.

### Exemplification

This invention is further illustrated by the following examples, which should not be construed as limiting.

### Example 1: Germline, somatic and clinical predictors of survival and adverse events following immunotherapy in a large real-world cohort

Possible confounding in retrospective data shown in **FIG. 1A** and FIG. 1B. **FIG. 1A** shows that disease burden can cause associations between OS and covariates. FIG. 1B shows that disease burden was adjusted for by imputing stage and ECOG status at treatment start for all patients from lab values and diagnosis codes in the full cohort of 60,000 patients. The predictors are highly significant and prognostic.

Controlling for disease burden explains many false associations with OS, as shown in **FIG. 2A and FIG. 2B. FIG. 2A** shows that controlling for disease burden, the number of associations with OS decreases, especially for lab values, which are easily influenced by disease burden. **FIG. 2B** shows the validation of associations with OS.

Predictive integrated interaction model shown in **FIG. 3A** **-** **FIG. 3C. FIG. 3A** shows the integration of all available features into a pheno-marker predicting OS. Baseline model includes age, gender, treatment, start year, line of treatment. Additional features include germline, somatic, labs, imputed ECOG/stage. **FIG. 3B and Fig. 3C** show a time dependent interaction analysis: being on ICIs significantly worse if predicted to be worse, and as shown in **FIG. 3B****,** predictor is prognostic; and as shown in **FIG. 3C****,** predictor is predictive).

ICI specific biomarkers for OS are shown in **FIG. 4A and FIG. 4B. FIG. 4A** shows that to find associations that are not just prognostic but predictive, one can do an interaction survival analysis with treatment type. **FIG. 4B** shows the many significant ICI-specific biomarkers, some previously found, such as TMB (Samstein et al. (2019) Nat. Genet. 51:202-206), SNV in PBRM1 (Miao et al. (2018) Science 359:801-806), and CDKN2A (Zhang et al. (2018) J. Clin. Oncol. 36:9102-9102).

Associations with irAEs are shown in **FIG. 5A** **-** **FIG. 5C****.** **FIG. 5A** **which** shows that to model irAEs, the disclosed model uses an additional state in survival model. This model takes care of competing hazards, which are important for irAE prediction. Many associations, as shown in **FIG. 5C****,** are found with irAEs, which allows building a preliminary predictor, which can identify significantly different incidence rate of irAEs, as shown in **FIG. 5B****.**

### Example 2: Germline predictors of immune-related adverse events

Data attributes related to 2,600 pan-cancer patients on immune checkpoint inhibitors are shown in **FIG. 6A and FIG. 6B****,** in which **FIG. 6A** shows the treatment data and **FIG. 6B** shows the cancer data. Tumor panel sequencing was used to identify germline features. Adverse Events (AE) were quantified from electronic medical records (EPIC).

A representative exemplary genome-wide association study to determine whether any single germline mutation associated with AE is shown in **FIG. 7A and Fig. 7B****.** The study tests each common germline variant (~1M in total) for association with an AE during treatment, and modeling competing risk of death. The results include multiple significant associations, and an ability to construct a "risk score."

Representative results for IL7 SNP association (**FIG. 8A**: graph; **FIG. 8C** and **FIG. 8D****:** table) are shown in FIG. 8A - FIG. 8D**.** FIG. 8A shows the distribution of effect sizes for this SNP with irAEs across cancer types. Significant effects (p-value<0.05) of this SNP on irAEs are observed across multiple cancer types. **FIG. 8C** shows the incidence of irAEs over time, stratified on the germline SNP allele. Germline SNP carriers exhibit a visibly higher incidence of irAEs over time (*i*.*e*., the SNP is predictive of an increased rate of irAEs). **Fig. 8D** shows the incidence in a replication cohort at another institution, a significant association with incidence is again observed.

Representative results for IL22RA1 SNP association (**FIG. 8B**: graph) are shown in **FIG. 8B. FIG. 8B** shows the distribution of effect sizes for this SNP with irAEs across cancer types. Significant effects (p-value<0.05) of this SNP on irAEs are observed across multiple cancer types.

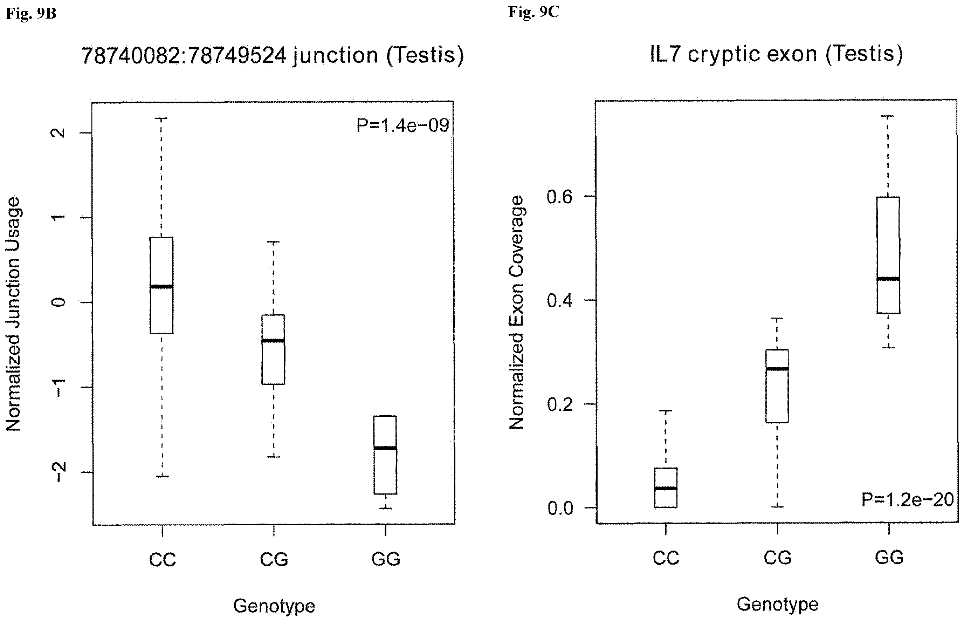

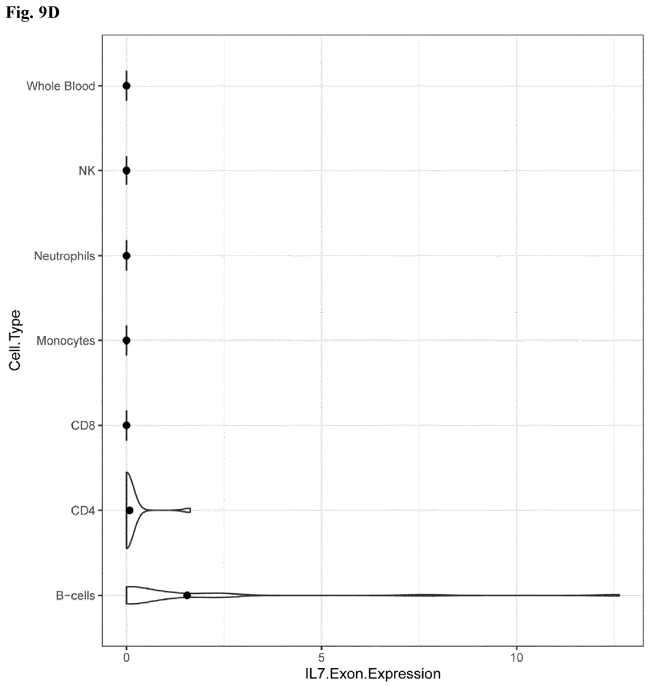

Representative results for effects of SNPs (*e.g*., rs7816685) on IL7 splicing are shown in FIG. 9A - FIG. 9D**.** **FIG. 9A** shows IL7 splicing in the testis tissue data from the GTEx consortium, stratified on the rs7816685 germline SNP allele. A significant increase in splicing to a cryptic exon overlapping the SNP is observed in allele carriers. This germline SNP is in the splice junction and likely driving increased splicing of this exon, which mediates it's effect on irAE incidence. **FIG. 9B** shows the association of this SNP with the canonical IL7 exon-exon junction observed in GTEx. **FIG. 9C** shows the association of this SNP with the novel cryptic exon. The association with the cryptic exon is much stronger, further supporting the model where this SNP influences IL7 activity through splicing of the cryptic exon. **FIG. 9D** shows the activity of the cryptic exon in publicly available immune cell data. The exon appears to be primarily active in B-cells.

### Incorporation by Reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

Also incorporated by reference in their entirety are any polynucleotide and polypeptide sequences which reference an accession number correlating to an entry in a public database, such as those maintained by The Institute for Genomic Research (TIGR) on the world wide web and/or the National Center for Biotechnology Information (NCBI) on the world wide web.

### Equivalents

Those skilled in the art will recognize, or be able to ascertain using no more than routine experimentation, many equivalents to the specific embodiments of the present invention described herein. Such equivalents are intended to be encompassed by the following claims.

Further optional embodiments are described in the following:
In an optional embodiment the disclosure relates to a method of identifying the likelihood of development of immune-related adverse events (irAE) in a subject due to immune checkpoint therapy, the method comprising:
a) obtaining or providing a subject sample from a subject having cancer;
b) measuring the amount of at least one germline biomarker listed in Table 1 in the subject sample; and
c) comparing said amount of the at least one germline biomarker listed in Table 1 to a control,
wherein the presence of or a significantly increased amount of the at least one germline biomarker listed in Table 1 in the subject sample, relative to the control, identifies the development of irAE due to immune checkpoint therapy as being more likely; and wherein the absence of or a significantly decreased amount of the at least one germline biomarker listed in Table 1 in the subject sample, relative to the control, identifies the development of irAE due to immune checkpoint therapy as being less likely.

In an optional embodiment the disclosure relates to a method as described above, further comprising recommending, prescribing, or administering the immune checkpoint therapy if the development of irAE due to immune checkpoint therapy is determined to be less likely or administering an anti-cancer therapy other than the immune checkpoint therapy if the development of irAE due to immune checkpoint therapy is determined to be more likely.

In an optional embodiment the disclosure relates to a method as described above, wherein the anti-cancer therapy is selected from the group consisting of targeted therapy, chemotherapy, radiation therapy, and/or hormonal therapy.

In an optional embodiment the disclosure relates to a method as described above, wherein the control is determined from a cancerous or non-cancerous sample from one or more members of the same species to which the subject belongs.

In an optional embodiment the disclosure relates to a method as described above, wherein the control is determined from a cancerous or non-cancerous sample representative of said one or more members who received immune checkpoint therapy but either did not develop irAE or developed irAE to an extent not exceeding a permissible level.

In an optional embodiment the disclosure relates to a method as described above" wherein the amount of the at least one germline biomarker listed in Table 1 is detected using a whole exome sequencing method.

In an optional embodiment the disclosure relates to a method as described above" wherein the amount of the at least one germline biomarker listed in Table 1 is detected by imputing it from its amount in a non-germline sample (*e.g*., tumor sample).

In an optional embodiment the disclosure relates to a method as described above" wherein the germline biomarker is a germline SNP.

In an optional embodiment the disclosure relates to a method as described above" wherein the subject sample and/or the control is selected from the group consisting of *ex vivo* and *in vivo* samples.

In an optional embodiment the disclosure relates to a method as described above" wherein the subject sample and/or the control is a portion of a single sample or pooled samples obtained from the subject.

In an optional embodiment the disclosure relates to a method as described above" wherein the subject sample and/or the control has not been contacted with any anti-cancer treatment or inhibitor of an immune checkpoint.

In an optional embodiment the disclosure relates to a method as described above, wherein the subject has not been administered any anti-cancer treatment or inhibitor of an immune checkpoint.

In an optional embodiment the disclosure relates to a method as described above" wherein the subject sample is selected from the group consisting of serum, whole blood, plasma, urine, cells, cell lines, and biopsies.

In an optional embodiment the disclosure relates to a method as described above" wherein the amount of the at least one germline biomarker listed in Table 1 is detected using a reagent which specifically binds with the germline biomarker.

In an optional embodiment the disclosure relates to a method as described above" wherein the reagent has a label group attached thereto, optionally wherein the label group comprising: a radioisotope, a fluorescent compound, an enzyme, or an enzyme co-factor.

In an optional embodiment the disclosure relates to a method as described above" wherein the at least one germline biomarker listed in Table 1 is assessed by liquid chromatography tandem mass spectrometry (LC-MS), HPLC, and/or mass spectrometry.

In an optional embodiment the disclosure relates to a method as described above" wherein the step of detecting further comprises purifying and/or concentrating the at least one germline biomarker listed in Table 1.

In an optional embodiment the disclosure relates to a method as described above, wherein the at least one germline biomarker listed in Table 1 is somatic CNA amplification TMPRSS2, somatic CNA amplification NSD1, somatic CNA amplification UIMC1, somatic CNA amplification FGFR4, somatic CNA amplification RUNX1, somatic CNA amplification TDG, CNA deletion WAS, CNA deletion ARAF, somatic SNV mutation in ARID1A, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), germline risk score for blood cell counts, germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs).

In an optional embodiment the disclosure relates to a method as described above" wherein the immune checkpoint therapy comprises an inhibitor of at least one selected from the group consisting of PD-1, PD-L1, PD-L2, TIM-3, LAG-3, CTLA-4, and combinations thereof.

In an optional embodiment the disclosure relates to a method as described above" wherein the inhibitor comprises at least one antibody selected from the group consisting of anti-PD-1 antibodies, anti-CTLA4 antibodies, anti-PD-L1 antibodies, anti-PD-L2 antibodies, and combinations thereof.

In an optional embodiment the disclosure relates to a method as described above" wherein the immune checkpoint therapy comprises an anti-PD-1 antibody.

In an optional embodiment the disclosure relates to a method as described above" wherein the cancer is selected from the group consisting of melanoma, bladder cancer, and renal cell cancer (RCC).

In an optional embodiment the disclosure relates to a method as described above, wherein the subject is a mammal.

In an optional embodiment the disclosure relates to a method as described above" wherein the mammal is an animal model of cancer.

In an optional embodiment the disclosure relates to a method as described above" wherein the mammal is a human or a rodent.

In an optional embodiment the disclosure relates to a method of selecting a subject having cancer for treatment with an immune checkpoint inhibitor (ICI), comprising:
a) measuring the amount of at least one germline biomarker listed in Table 1 in the subject sample;
b) comparing said amount of the at least one germline biomarker listed in Table 1 to a control; and
c) selecting the subject for treatment with an ICI if the at least one germline biomarker listed in Table 1 is absent or has a significantly decreased amount in the subject sample relative to the control.

In an optional embodiment the disclosure relates to a, method of selecting a subject having cancer for treatment with an anti-cancer therapy other than the immune checkpoint therapy, comprising:
a) measuring the amount of at least one germline biomarker listed in Table 1 in the subject sample;
b) comparing said amount of the at least one germline biomarker listed in Table 1 to a control; and
c) selecting the subject for treatment with an anti-cancer therapy other than the immune checkpoint therapy if the at least one germline biomarker listed in Table 1 is present or has a significantly increased amount in the subject sample relative to the control.

In an optional embodiment the disclosure relates to a method of treating a subject having cancer with an immune checkpoint inhibitor (ICI), comprising:
a) selecting a subject for treatment with an ICI, wherein the subject has been determined to have at least one germline biomarker listed in Table 1 that is absent or has a significantly decreased amount in the subject sample relative to a control; and
b) administering an ICI to the subject.

In an optional embodiment the disclosure relates to a method of treating a subject having cancer with an anti-cancer therapy other than the immune checkpoint therapy, comprising:
a) selecting a subject for treatment with an anti-cancer therapy other than the immune checkpoint therapy, wherein the subject has been determined to have at least one germline biomarker listed in Table 1 that is present or has a significantly increased amount in the subject sample relative to a control; and
b) administering an anti-cancer therapy other than the immune checkpoint therapy to the subject.

In an optional embodiment the disclosure relates to a method as described above" wherein the anti-cancer therapy is selected from the group consisting of targeted therapy, chemotherapy, radiation therapy, and/or hormonal therapy.

In an optional embodiment the disclosure relates to a method as described above" wherein the control is determined from a cancerous or non-cancerous sample from one or more members of the same species to which the subject belongs.

In an optional embodiment the disclosure relates to a method as described above" wherein the control is determined from a cancerous or non-cancerous sample representative of said one or more members who received immune checkpoint therapy but either did not develop irAE or developed irAE to an extent not exceeding a persmissible level.

In an optional embodiment the disclosure relates to a method as described above" wherein the amount of the at least one germline biomarker listed in Table 1 is detected using a whole exome sequencing method.

In an optional embodiment the disclosure relates to a method as described above" wherein the amount of the at least one germline biomarker listed in Table 1 is detected by imputing it from its amount in a non-germline sample (*e.g*., tumor sample).

In an optional embodiment the disclosure relates to a method as described above" wherein the germline biomarker is a germline SNP.

In an optional embodiment the disclosure relates to a method as described above" wherein the subject sample and/or the control is selected from the group consisting of *ex vivo* and *in vivo* samples.

In an optional embodiment the disclosure relates to a method as described above" wherein the subject sample and/or the control is a portion of a single sample or pooled samples obtained from the subject.

In an optional embodiment the disclosure relates to a method as described above" wherein the subject sample and/or the control has not been contacted with any anti-cancer treatment or inhibitor of an immune checkpoint.

In an optional embodiment the disclosure relates to a method as described above" wherein the subject has not been administered any anti-cancer treatment or inhibitor of an immune checkpoint.

In an optional embodiment the disclosure relates to a method as described above" wherein the subject sample is selected from the group consisting of serum, whole blood, plasma, urine, cells, cell lines, and biopsies.

In an optional embodiment the disclosure relates to a method as described above, wherein the amount of the at least one germline biomarker listed in Table 1 is detected using a reagent which specifically binds with the germline biomarker.

In an optional embodiment the disclosure relates to a method as described above" wherein the reagent has a label group attached thereto, optionally wherein the label group comprising: a radioisotope, a fluorescent compound, an enzyme, or an enzyme co-factor.

In an optional embodiment the disclosure relates to a method as described above" wherein the at least one germline biomarker listed in Table 1 is assessed by liquid chromatography tandem mass spectrometry (LC-MS), HPLC, and/or mass spectrometry.

In an optional embodiment the disclosure relates to a method as described above" wherein the step of detecting further comprises purifying and/or concentrating the at least one germline biomarker listed in Table 1.

In an optional embodiment the disclosure relates to a method as described above, wherein the at least one germline biomarker listed in Table 1 is somatic CNA amplification TMPRSS2, somatic CNA amplification NSD1, somatic CNA amplification UIMC1, somatic CNA amplification FGFR4, somatic CNA amplification RUNX1, somatic CNA amplification TDG, CNA deletion WAS, CNA deletion ARAF, somatic SNV mutation in ARID1A, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), germline risk score for blood cell counts, germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs).

In an optional embodiment the disclosure relates to a method as described above" wherein the immune checkpoint therapy comprises an inhibitor of at least one selected from the group consisting of PD-1, PD-L1, PD-L2, TIM-3, LAG-3, CTLA-4, and combinations thereof.

In an optional embodiment the disclosure relates to a method as described above" wherein the inhibitor comprises at least one antibody selected from the group consisting of anti-PD-1 antibodies, anti-CTLA4 antibodies, anti-PD-L1 antibodies, anti-PD-L2 antibodies, and combinations thereof.

In an optional embodiment the disclosure relates to a method as described above" wherein the immune checkpoint therapy comprises an anti-PD-1 antibody.

In an optional embodiment the disclosure relates to a method as described above" wherein the cancer is selected from the group consisting of melanoma, bladder cancer, and renal cell cancer (RCC).

In an optional embodiment the disclosure relates to a method as described above" wherein the subject is a mammal.

In an optional embodiment the disclosure relates to a method as described above" wherein the mammal is an animal model of cancer.

In an optional embodiment the disclosure relates to a method as described above" wherein the mammal is a human or a rodent.

## Claims

1. A method of identifying the likelihood of development of immune-related adverse events (irAE) in a subject due to immune checkpoint therapy, the method comprising:
a) obtaining or providing a subject sample from a subject having cancer;
b) measuring the amount of at least one germline biomarker in the subject sample, wherein the at least one germline biomarker is selected from:
germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs), CNA deletion WAS, CNA deletion ARAF, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), and germline risk score for blood cell counts; and
c) comparing said amount of the at least one germline biomarker to a control, thereby identifying the likelihood of development of immune-related adverse events (irAE) in the subject due to immune checkpoint therapy,
wherein the presence of or a significantly increased amount of the at least one germline biomarker in the subject sample, relative to the control, identifies the development of irAE due to immune checkpoint therapy as being more likely; and wherein the absence of or a significantly decreased amount of the at least one germline biomarker in the subject sample, relative to the control, identifies the development of irAE due to immune checkpoint therapy as being less likely, optionally
further comprising recommending, prescribing, or administering the immune checkpoint therapy if the development of irAE due to immune checkpoint therapy is determined to be less likely or administering an anti-cancer therapy other than the immune checkpoint therapy if the development of irAE due to immune checkpoint therapy is determined to be more likely.

2. A method of selecting a subject having cancer for treatment with an immune checkpoint inhibitor (ICI), comprising:
a) measuring the amount of at least one germline biomarker in the subject sample, wherein the at least one germline biomarker is selected from:
germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs), CNA deletion WAS, CNA deletion ARAF, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), and germline risk score for blood cell counts;
b) comparing said amount of the at least one germline biomarker to a control; and
c) selecting the subject for treatment with an ICI if the at least one germline biomarker is absent or has a significantly decreased amount in the subject sample relative to the control, thereby selecting the subject having cancer for treatment with the ICI.

3. A method of selecting a subject having cancer for treatment with an anti-cancer therapy other than an immune checkpoint therapy, comprising:
a) measuring the amount of at least one germline biomarker in the subject sample, wherein the at least one germline biomarker is selected from:
germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs), CNA deletion WAS, CNA deletion ARAF, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), and germline risk score for blood cell counts;
b) comparing said amount of the at least one germline biomarker to a control; and
c) selecting the subject for treatment with an anti-cancer therapy other than an immune checkpoint therapy if the at least one germline biomarker is present or has a significantly increased amount in the subject sample relative to the control, thereby selecting the subject having cancer for treatment with the anti-cancer therapy other than an immune checkpoint therapy.

4. A method of treating a subject having cancer with an immune checkpoint inhibitor (ICI), comprising:
a) selecting a subject for treatment with an ICI, wherein the subject has been determined to have at least one germline biomarker that is absent or has a significantly decreased amount in the subject sample relative to a control, wherein the at least one germline biomarker is selected from:
germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs), CNA deletion WAS, CNA deletion ARAF, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), and germline risk score for blood cell counts; and
b) administering an ICI to the subject, thereby treating the subject having cancer with the ICI.

5. A method of treating a subject having cancer with an anti-cancer therapy other than an immune checkpoint therapy, comprising:
a) selecting a subject for treatment with an anti-cancer therapy other than an immune checkpoint therapy, wherein the subject has been determined to have at least one germline biomarker that is present or has a significantly increased amount in the subject sample relative to a control, wherein the at least one germline biomarker is selected from:
germline variant near the IL7 gene or influencing IL7 splicing (SNP rsid rs7816685, or SNP rsid rs16906115, or other correlated SNPs), germline variant near the IL22RA1 gene (SNP rsid rs75824728 or correlated SNPs), CNA deletion WAS, CNA deletion ARAF, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), and germline risk score for blood cell counts; and
b) administering an anti-cancer therapy other than the immune checkpoint therapy to the subject, thereby treating the subject having cancer with the anti-cancer therapy other than an immune checkpoint therapy.

6. The method of any one of claims 1 to 5, wherein the anti-cancer therapy is selected from the group consisting of targeted therapy, chemotherapy, radiation therapy, and/or hormonal therapy.

7. The method of any one of claims 1 to 6, wherein the control is determined from a cancerous or non-cancerous sample from one or more members of the same species to which the subject belongs, optionally wherein the control is determined from a cancerous or non-cancerous sample representative of said one or more members who received immune checkpoint therapy but either did not develop irAE or developed irAE to an extent not exceeding a persmissible level.

8. The method of any one of claims 1 to 7, wherein the amount of the at least one germline biomarker is detected
a) using a whole exome sequencing method;
b) using a reagent which specifically binds with the germline biomarker, optionally wherein the reagent has a label group attached thereto, optionally wherein the label group comprising: a radioisotope, a fluorescent compound, an enzyme, or an enzyme co-factor;
c) by liquid chromatography tandem mass spectrometry (LC-MS), HPLC, and/or mass spectrometry; and/or
d) with a further step of purifying and/or concentrating the at least one germline biomarker.

9. The method of any one of claims 1 to 8, wherein the amount of the at least one germline biomarker is detected by imputing it from its amount in a non-germline sample (*e.g*., tumor sample).

10. The method of any one of claims 1 to 9, wherein the subject sample and/or the control
a) is selected from the group consisting of *ex vivo* and *in vivo* samples;
b) is a portion of a single sample or pooled samples obtained from the subject;
c) has not been contacted with any anti-cancer treatment or inhibitor of an immune checkpoint; and/or
d) is selected from the group consisting of serum, whole blood, plasma, urine, cells, cell lines, and biopsies.

11. The method of any one of claims 1 to 10, wherein the subject has not been administered any anti-cancer treatment or inhibitor of an immune checkpoint.

12. The method of any one of claims 1 to 11, wherein the at least one germline biomarker further comprises one or more additional germline biomarkers selected from: somatic CNA amplification TMPRSS2, somatic CNA amplification NSD1, somatic CNA amplification UIMC1, somatic CNA amplification FGFR4, somatic CNA amplification RUNX1, somatic CNA amplification TDG, CNA deletion WAS, CNA deletion ARAF, somatic SNV mutation in ARID1A, SNV mutation in ARID1B, SNV mutation in PIK3R1, SNV mutation in APC, SNV mutation in SETD2, SNV mutation in B2M, SNV mutation in BCOR, SNV mutation in CASP8, SNV mutation in KDM6A, SNV mutation in MSH6, SNV mutation in ROS1, germline risk score for bladder cancer, germline risk score for medication use (including anti-inflammatory or immunosuppressant medications), germline risk score for blood cell counts, germline variant near the IL7 gene or influencing IL7 splicing (SNP rsid rs7816685, or SNP rsid rs16906115, or other correlated SNPs), and germline variant near the IL22RA1 gene (SNP rsid rs75824728 or correlated SNPs).

13. The method of any one of claims 1 to 12, wherein the immune checkpoint therapy comprises an inhibitor of at least one selected from the group consisting of PD-1, PD-L1, PD-L2, TIM-3, LAG-3, CTLA-4, and combinations thereof, optionally wherein the inhibitor is an antibody.

14. The method of any one of claims 1 to 13, wherein the cancer is selected from the group consisting of melanoma, bladder cancer, and renal cell cancer (RCC).

15. The method of any one of claims 1 to 14, wherein the subject is an animal model of cancer, a human, or a rodent.
